# EUROPEAN PATENT SPECIFICATION

(11) **EP 2 329 018 B1**
(45) Date of publication and mention of the grant of the patent: **10.09.2014**
(21) Application number: 08750823.0
(22) Date of filing: 25.04.2008
(51) Int. Cl.: C12N 15/09, G06F 17/30, G06F 19/00

(54) **METHOD FOR THE ANNOTATION OF NATURAL PRODUCT GENE-CLUSTERS AND FOR THE GENERATION OF NOVEL BIOLOGICALLY ACTIVE CHEMICAL ENTITIES FROM DNA SEQUENCES IN SILICO**
VERFAHREN ZUR KOMMENTIERUNG VON NATURPRODUKT-GENCLUSTERN UND ZUR ERZEUGUNG NEUARTIGER BIOLOGISCH AKTIVER CHEMISCHER STOFFE AUS DNA-SEQUENZEN IN SILICO
PROCÉDÉ POUR L'ANNOTATION DE GROUPES DE GÈNES DE PRODUIT NATUREL ET POUR LA GÉNÉRATION DE NOUVELLES ENTITÉS CHIMIQUES BIOLOGIQUEMENT ACTIVES À PARTIR DE SÉQUENCES D'ADN IN SILICO

(43) Date of publication of application: 08.06.2011
(73) Proprietor: GenQuad AG, 8808 Pfäffikon (CH)
(72) Inventor: STARCEVIC, Antonio, 10000 Zagreb (HR); ZUCKO, Jurica, 10000 Zagreb (HR); SIMUNKOVIC, Jurica, 10000 Zagreb (HR); LONG, Paul, F., London N7 0ET (GB); CULLUM, John, 167727 Lohnsfeld (DE); HRANUELI, Daslav, 10000 Zagreb (HR)
(74) Representative: Dragun, Tihomir
(86) International application number: PCT/HR2008/000012
(87) International publication number: WO 2009/130520

(56) References cited:
- WO-A-03/014312
- TAE HONGSEOK ET AL: "ASMPKS: an analysis system for modular polyketide synthases." BMC BIOINFORMATICS 2007, vol. 8, 2007, page 327, XP002517614 ISSN: 1471-2105 cited in the application
- ANSARI MOHD ZEESHAN ET AL: "NRPS-PKS: a knowledge-based resource for analysis of NRPS/PKS megasynthases." NUCLEIC ACIDS RESEARCH 1 JUL 2004, vol. 32, no. Web Server issue, 1 July 2004 (2004-07-01), pages W405-W413, XP002517615 ISSN: 1362-4962
- YADAV GITANJALI ET AL: "SEARCHPKS: A program for detection and analysis of polyketide synthase domains." NUCLEIC ACIDS RESEARCH 1 JUL 2003, vol. 31, no. 13, 1 July 2003 (2003-07-01), pages 3654-3658, XP002517616 ISSN: 1362-4962 cited in the application
- YADAV ET AL: "Computational Approach for Prediction of Domain Organization and Substrate Specificity of Modular Polyketide Synthases" JOURNAL OF MOLECULAR BIOLOGY, LONDON, GB, vol. 328, no. 2, 25 April 2003 (2003-04-25), pages 335-363, XP005472710 ISSN: 0022-2836 cited in the application
- MORIYA YUKI ET AL: "KAAS: an automatic genome annotation and pathway reconstruction server." NUCLEIC ACIDS RESEARCH JUL 2007, vol. 35, no. Web Server issue, July 2007 (2007-07), pages W182-W185, XP002517617 ISSN: 1362-4962
- WU JIANMIN ET AL: "KOBAS server: a web-based platform for automated annotation and pathway identification." NUCLEIC ACIDS RESEARCH 1 JUL 2006, vol. 34, no. Web Server issue, 1 July 2006 (2006-07-01), pages W720-W724, XP002517618 ISSN: 1362-4962
- KARP P D ET AL: "Integrated pathway-genome databases and their role in drug discovery" TRENDS IN BIOTECHNOLOGY, ELSEVIER PUBLICATIONS, CAMBRIDGE, GB, vol. 17, no. 7, 1 July 1999 (1999-07-01), pages 275-281, XP004169726 ISSN: 0167-7799
- WEISSMAN KIRA J ET AL: "Protein-protein interactions in multienzyme megasynthetases." CHEMBIOCHEM : A EUROPEAN JOURNAL OF CHEMICAL BIOLOGY 14 APR 2008, vol. 9, no. 6, 14 April 2008 (2008-04-14), pages 826-848, XP002517619 ISSN: 1439-7633
- GONZÁLEZ-LERGIER JOANNA ET AL: "Theoretical considerations and computational analysis of the complexity in polyketide synthesis pathways." JOURNAL OF THE AMERICAN CHEMICAL SOCIETY 13 JUL 2005, vol. 127, no. 27, 13 July 2005 (2005-07-13), pages 9930-9938, XP002517620 ISSN: 0002-7863
- WISTRAND MARKUS ET AL: "Improved profile HMM performance by assessment of critical algorithmic features in SAM and HMMER." BMC BIOINFORMATICS 2005, vol. 6, 2005, page 99, XP002517621 ISSN: 1471-2105
- SHERMAN DAVID H ET AL: "Clearing the skies over modular polyketide synthases" ACS CHEMICAL BIOLOGY, vol. 1, no. 8, 2006, pages 505-509, XP002517622 ISSN: 1554-8929(print) 1554-8937(ele
- STARCEVIC ANTONIO ET AL: "ClustScan: an integrated program package for the semi-automatic annotation of modular biosynthetic gene clusters and in silico prediction of novel chemical structures" NUCLEIC ACIDS RESEARCH, vol. 36, no. 21, December 2008 (2008-12), pages 6882-6892, XP002517623 ISSN: 0305-1048

## Description

### Technical Field:

The present invention relates to establishing an *in silico* method that allows rapid, semi-automatic annotation of modular biosynthetic gene-clusters starting from DNA sequences and for the generation of novel biologically active chemical entities. Potential protein coding regions and domains are identified by programs running on a Linux server and the results are presented to the user in a graphical interface with a *Java* client program. The user can edit the results and assemble biosynthetic gene-clusters. The activity and specificity of microbial natural product catalytic domains is predicted using published data about functional amino acid residues as well as new analyses carried out during development of the method. The predictions are used to deduce the chemical structure of the linear microbial natural products produced by the catalytic domains encoded by the gene-cluster, as well as making a prediction of the cyclic structure. The method also allows the generation of a library of virtual novel chemical compounds obtained by the *in silico* homologous recombination of sequenced modular biosynthetic gene-clusters. This library is used for *in silico* screening to discover potential therapeutic agents using Computer aided Drug Design (CDD) technology.

### Background of the Invention (Prior art):

Many important secondary metabolites in bacteria are synthesised by modular biosynthetic gene-clusters: polyketide synthases (PKS), non-ribosomal peptide synthetases (NRPS), NRPS-independent siderophore synthetases (NIS) and hybrid synthase/synthetase gene-clusters. These include polyketide antibiotics (e.g. erythromycin), immuno-suppressants (e.g. rapamycin), antiparasitic agents (e.g. avermectin), as well as peptide antibiotics (e.g. vancomycin), immuno-suppressants (e.g. cyclosporin) and herbicides (e.g. bialaphos). There are also hybrid gene-clusters that encode both PKS and NRPS modules producing anti-tumour agents such as bleomycin and epothilone (see references in: Weissman & Leadlay, Nat. Rev. Microbiol., 3, 925-936, 2005.; Finking & Marahiel, Ann. Rev. Microbiol., 58, 453-488, 2004.; Hranueli et al., Curr. Med. Chem., 12, 1697-1704, 2005; Challis, ChemBioChem, 6, 601-611, 2005).

Large scale DNA sequencing has revealed many modular gene-clusters, whose products are not known (Bentley et al., Nature, 417, 141-147, 2002; Ikeda et al., Nat. Biotechnol. 21, 526-531, 2003; Oliynyk et al., Nat. Biotechnol., 25, 447-453, 2007). The genome sequencing of bacteria is expanding rapidly with over 500 genome sequences publicly available at present. Current advances in sequencing technology will lead to an explosive increase in known genome sequences in the near future. Moreover, recent developments in DNA sequencing technology have opened up new opportunities allowing the DNA sequencing of unculturable microorganisms. This field of study, called metagenomic, allows access to the genetic potential of culturable and unculturable microorganisms (see references in: Dunlap et al., Curr. Med. Chem., 13, 697-710, 2006). For example, in 2004 the Craig Venter's Institute launched the Sorcerer II global ocean sampling expedition to evaluate the microbial diversity in the world's oceans using molecular tools and techniques originally developed to sequence the human genome. Their data represents the largest metagenomic dataset ever put into the public domain with more than 7.7 million sequencing reads comprising 6.3 billion base pairs of DNA (Rusch et al., PLoS Biol. 5, e77, 2007). There are several hundred sequenced modular gene-clusters in public databases that have not been adequately analysed and their number will grow exponentially with every new bacterial genome and metagenome sequenced. Traditional screening approaches will miss many interesting compounds, because they are not produced under the fermentation conditions used. Additionally, purification of an unknown compound is costly and time-intensive. Gene-clusters encoding secondary metabolic biosynthetic pathways can be recognised by bioinformatic methods and allow partial prediction of the nature of the compounds produced. Computational tools are now needed to rapidly mine such massive datasets to hunt for gene-clusters encoding entirely new compounds which could be the medicines of the future.

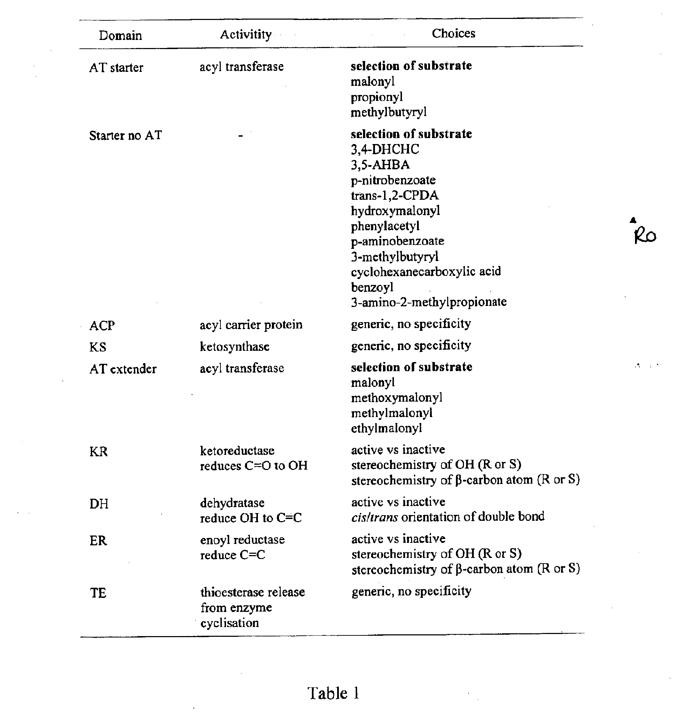

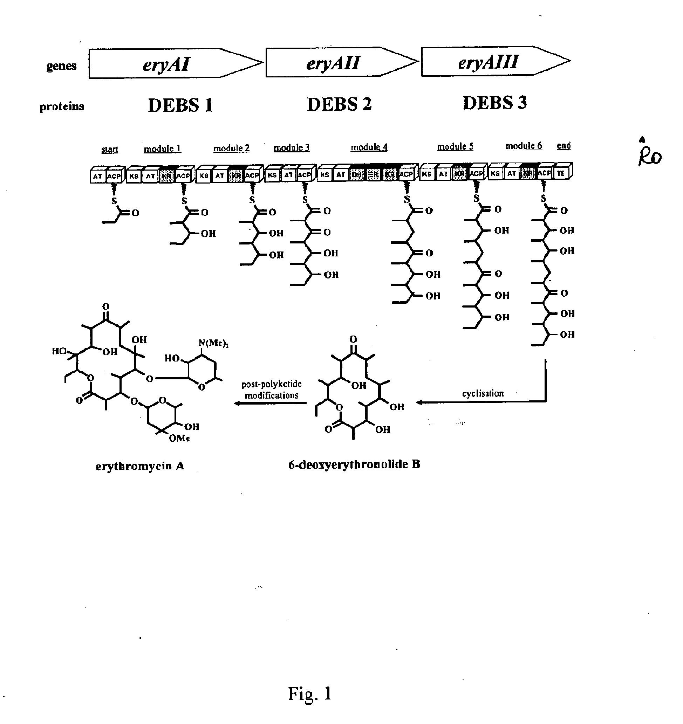

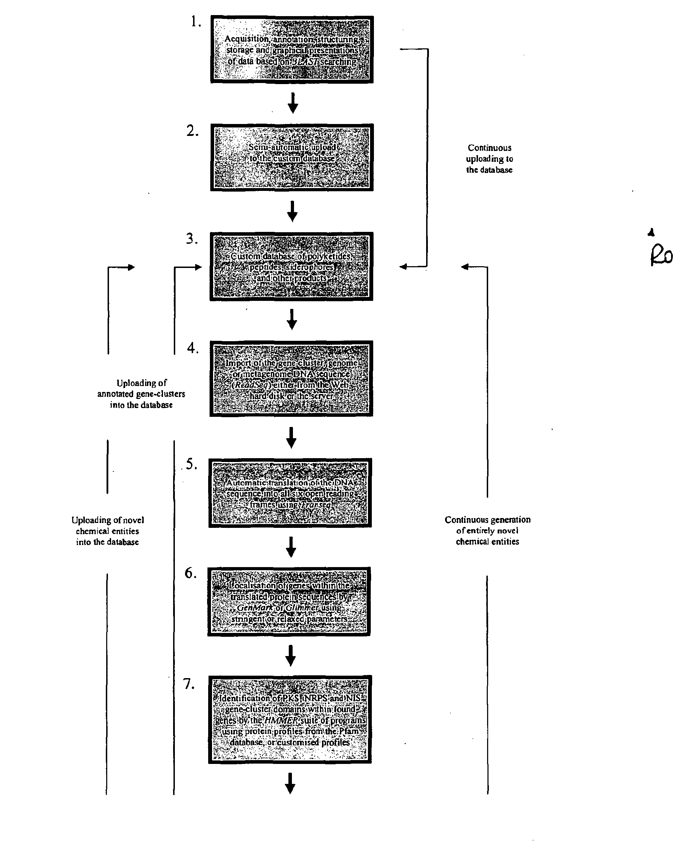

The biosynthesis of these secondary metabolites occurs by a series of sequential steps and each step is encoded by a group of catalytic domains grouped as different modules. The modules are usually arranged across several polypeptides, with often more than one module in a polypeptide. For example, in the case of PKS modules, there are a series of domains that direct different stages of each biosynthetic step. The acyl transferase domain (AT) links the new extender unit to the acyl carrier protein (ACP) domain. The ketosynthetase domain (KS) then transfers the growing chain to the new extender unit and performs condensation. The basic module thus needs only KS-AT-ACP and will build a keto group into the chain. However, many modules contain further reduction domains. If a ketoreductase (KR) domain is present, the keto group will be reduced to a hydroxyl group. If there is also a dehydratase domain (DH) the hydroxyl group will be reduced to a double bond and if there is also an enoyl reductase domain, the keto-group will be reduced completely (Fig 1). This means that if such systems could be programmed at will, there would be a large number of possible compounds (e.g. if there were only 10 modules there would be about 10⁹ compounds possible). During the last decade or so, scientists have started to modify these multi-modular *enzymes in vitro* using genetic engineering technology called combinatorial biosynthesis. By inactivating individual domains, or adding active domains from other gene-clusters, they have generated novel polyketides with differently reduced β-carbons and different stereochemistry. They have also swapped, deleted or added individual domains and entire modules, in order to synthesise novel polyketides with smaller or larger polyketide aglycons. However, this approach has its own limitations. It requires specific expertise and is time consuming. Because of this, *in vitro* genetic manipulations have lead to the synthesis of only about 200 novel polyketides (Weissman and Leadlay, Nat. Rev. Microbiol., 3,925-936, 2005). The majority of gene-clusters constructed in this way do not produce any compound or their compound yields are extremely low and unsuitable for commercial production (see references in Khosla & Keasling. Nat. Rev. Drug Discov., 2, 1019-1025, 2003). Because of this, the construction of novel gene-clusters *by in vivo* homologous recombination between pairs of existing clusters was proposed (Hranueli et al., Curr. Med. Chem., 12, 1697-1704, 2005). Such an approach will allow recombination amongst DNA sequences in a similar way to that which occurs in nature during the evolution of new clusters. This will hopefully greatly reduce the occurrence of non-functional DNA junctions that are common after recombination *in vitro* (Hranueli et al., Curr. Med. Chem., 12, 1697-1704, 2005). The largest number of known polyketides are synthesised by species of *Streptomyces* and related genera. Streptomycetes are soil dwelling bacteria with large linear chromosomes that can occasionally form structures of two joined chromosomes in inverted orientation (e.g. Pandza et al., Mol. Microbiol., 28, 1165-1176, 1998; Wenner et al., Mol. Microbiol. 50, 411-425, 2003; Denapaite et al., Food Technol. Biotechnol., 43, 9-17, 2005; Petkovic et al., Microbiol. Mol. Biol. R. 70, 704-728, 2006). There are already three genomes sequenced and available in public databases (Bentley et al., Nature, 417, 141-147, 2002; Ikeda et al., Nat. Biotechnol. 21, 526-531, 2003; Oliynyk et al., Nat. Biotechnol., 25, 447-453, 2007). Because the molecular genetics *of Streptomyces* species is well developed it should be possible to construct desired recombinants *in vivo* to obtain the desired products (Kieser et al., "Practical Streptomyces Genetics, 2nd Edition", John Innes Foundation 2002, ISBN 0-7084-0623-8).

Homologous recombination systems are ubiquitous in bacteria. Recombination efficiency depends on the degree of homology between the sequences and usually drops off very rapidly, when the homology is less than 100%. Little is known about the details of recombination in *Streptomyces* species. *Streptomyces* species possess a *recA* gene (Bentley et al., Nature, 417, 141-147, 2002; Ikeda et al., Nat. Biotechnol. 21, 526-531, 2003; Oliynyk et al., Nat. Biotechnol., 25, 447-453, 2007), but little is known of other recombination genes. The genome sequences of *S*. *coelicolor* A3(2) (Bentley et al., Nature, 417, 141-147, 2002) and S. *avermitilis* (Ikeda et al., Nat. Biotechnol. 21, 526-531, 2003) do not show a detectable *recBCD* homologue. The RecBCD enzyme is important for *E. coli* recombination as it provides a combination of DNA helicase and single-strand endonuclease activity, which probably provides a single stranded substrate for strand invasion (Dillingham et al., Nature, 432, 187-193, 2004). A homologue is present in *Mycobacterium tuberculosis* (Springer et al., Mol. Microbiol., 53, 1601-1609, 2004), which is related to *Streptomyces.* In some other Gram-positive bacteria, there is an alternative enzyme with similar properties, but which has little sequence conservation with RecBCD (e.g. the AddAB enzyme of *Bacillus subtilis).* There are also no detectable *addAB* homologues in the *Streptomyces* genome sequences, but there are four possible DNA helicase genes, one of which may encode an enzyme with similar activities. Both RecBCD and AddAB respond to chi-sequences, which are short asymmetric purine rich sequences. When the progressing enzyme encounters a chi-sequence, the endonuclease activity is switched off so that the helicase produces single stranded DNA (Chedin et al., Mol. Microbiol., 29, 1369-1377, 1998). Chi-sequences stimulate recombination. *Streptomyces* and other actinomycetes do not have homologues of the mismatch repair genes *mutSL,* which are present in most other prokaryotes and eukaryotes. In *E. coli* it was shown that the mismatch repair system greatly reduces recombination between sequences that have sequence divergence *(*Rayssiguier et al., Nature, 342, 396-401, 1989) so that it is possible that recombination between such sequences in *Streptomyces* will be more efficient than in most other bacteria. The RecA protein promotes pairing of a single stranded DNA end with double stranded DNA; efficient pairing needs a minimal length of exact sequence match and this minimal efficient pairing (MEPS) sequence is estimated as about 27 bp (Shen and Huang, Genetics, 112, 441-457 1986). A high degree of sequence similarity in the region around the MEPS is probably also necessary and it is suggested that a sequence identity of about 78% is needed (Datta et al., Proc. Natl. Acad. Sci. USA., 94, 9757-9762, 1997).

When a novel chemical entity is available either *in vivo,* or *in silico,* Computer aided Drug Design (CDD) can be to simulate receptor-drug interactions. In virtual high throughput screening, a target can be screened against a database of small molecules to see which binds strongly to the target. Several million compounds can be screened in a matter of days with today's powerful computational resources, saving considerable time and expense. Prediction of Activity Spectra for Substances (PASS) software is a good example of a virtual high throughput screening product (see references in: Stepanchikova et al. Curr. Med. Chem., 10, 225-233, 2003), with a web-site internet version introduced in December 2001 (http://www.ibmh.msk.su/PASS). There are a variety of methods used in these similarity searches. Shape similarity seeks compounds with structure similar to known actives, while shape complementarity is the basis of most receptor-based design, where the goal is to identify compounds complementary in shape to a given receptor. However, drug-receptor interactions occur at the atomic level and the biochemical and biophysical properties of both the drug and target must be considered to understand how drugs bind to protein receptors. Predicting physicochemical properties, such as hydrophobicity and polarity is key to lead optimisation (see references in: Zauhar et al. J. Med. Chem., 46, 5674-5690, 2003).

In principle it should be possible to predict the biosynthetic activity of a module from the DNA sequence that encodes it. The choice of extender unit seems to be governed by the AT domain and differences between domains that choose malonyl-CoA, methoxymalonyl-CoA, methylmalonyl-CoA and ethylmalonyl-CoA have been recognised. In addition to AT domains in extender modules, there are often AT domains in starter modules or loading domains. A set of AT starter domains that incorporate malonyl, propionyl and methylbutyryl starters have also been recognised. There are also at least 10 known unusual loading domains where AT domains are not present. Nevertheless, amino acid sequences in loading domains responsible for the choice of unusual starters are also recognised. It is also possible to recognise the reduction domains (KR, DH and ER) by homology and, thus, predict the degree of the reduction of the α- carbon and the stereochemistry of both α- and β-carbons. This is complicated by the fact that sometimes non-active reduction domains are present. The KR domain is the best characterised domain in terms of structural determination of differential activities. Active KR domains determine the chirality of the hydroxyl product and bioinformatic analysis identified amino acid residues involved in this choice (Caffrey, ChemBioChem., 4:649-662, 2003). Bioinformatics also suggested that KR rather than KS determined the stereochemistry of β-carbon groups, when C3 or C4 units are incorporated (Starcevic et al., ChemBioChem, 8, 28-31, 2007). A comparison of 3-D structures of two KR domains of different specificity gave more detailed information on amino acid residues involved in determining both hydroxyl and β-carbon stereochemistry (Keatinge-Clay, Chemistry & Biology, 14, 898-908, 2007). There are six possible products (A1, A2, B1, B2, C1, C2), which correspond to three possible ketoreduction outcomes (either hydroxyl stereoisomer - A or B, or no reduction C) coupled with two β-carbon chiralities (called 1 and 2). For DH and ER domains, the prediction should firstly distinguish active and inactive domains. It is also known the DH domains are involved in the *cis*/*trans* orientation at the double bond and the ER domains of the stereochemistry of α- and β-carbons if the double bond is formed. However, insufficient structural information is available to make predictions based on knowledge of function alone.

NRPS and NIS gene-clusters have a similar organisation to PKS gene-clusters, but the modules assemble amino acids (see references in: Finking & Marahiel, Ann. Rev. Microbiol., 58, 453-488, 2004; Challis, ChemBioChem, 6, 601-611, 2005). The modules are composed of catalytic domains with standard (condensation, adenylation and peptidyl carrier protein; C-A-PCP) domains present in all modules as well as optional domains (e.g. epimerization (Epi), cylization (Cyc), N-methylation (N-Meth), domains etc.). In addition to the 20 amino acids present in most proteins, NRPSs may catalyse the formation of polypeptides composed of other (more than 400) amino acids. There is, thus, much potential for combinatorial biosynthesis.

A tool that helps the analysis of new gene-clusters is the *NRPS-PKS database (SEARCHPKS;* Yadav et. al, Nucleic Acids Res., 31, 3654-3658, 2003; http://www.nii.res.in/ nrps-pks.html), which holds data on PKS and NRPS gene-clusters including module and domain structure and chemical structures of the gene-cluster products. It allows users to input protein sequences to be used in BLAST (Altschul et al., J. Mol. Biol., 215, 403-410, 1990) searches to identify domains and find the closest sequences in the database. This allows prediction of whether an AT-domain uses malonyl-CoA or methylmalonyl-CoA as a substrate (i.e. whether a C2 or C3 unit is incorporated into the polyketide). Within the *ASMPKS database* (Tae et al., BMC Bioinformatics, 8, 327, 2007; http://gate.smallsoft.co.kr:8008/ %7Ehstae/asmpks/index.html) there is a tool *MAPSI* that uses a similar analysis methodology, but integrates it with a graphical display that shows the presence of domains in genes so that modules can be easily recognised. It also allows the display of a predicted linear polyketide chain product for which the user has to select starter and extender units from the list. However, the stereochemistry of the product is not considered. The company ECOPIA has also developed a software tool *DecipherIT™* (Zazopoulos et al., Nat. Biotechnol., 21, 187-190, 2003), which helps annotation of new gene-clusters based on comparison with a database of known gene-clusters. A slightly different tool is the *Biogenerator* program (Zotchev et al., J. Med. Chem., 49, 2077-2087, 2006), which allows the construction of new polyketides based on known *modules in silico.* The programs listed above all depend on comparison with sequences in a database to analyse new gene-clusters. This works best for gene-clusters closely related to well-characterised gene-clusters. However, almost all of them are using predictions based on similarity searches and can be considered rudimentary.

However, in the last few years there has also been considerable published work to identify protein features responsible for specificity of domains (Table 1). Amino acid residues in AT domains that differ between malonyl-CoA-incorporating and methylmalonyl-CoA-incorporating domains have been identified from multiple alignments of AT sequences (Yadav et. al, J. Mol. Biol., 328, 335-363, 2003). Specific residues in the ketoreductase (KR) domain that seem to be responsible for the stereochemistry of hydroxyl groups and of the β-carbon atom in the extender unit have also been identified (Caffrey, ChemBioChem, 4, 654-657, 2003; Starcevic et al., ChemBioChem, 8, 28-31, 2007; Keatinge-Clay, Chemistry & Biology, 14, 898-908, 2007; Castonguay et al., J. Am. Chem. Soc., 129, 13758-13769, 2007). A complication in the analysis of modules is that reduction domains (i.e. KR, DH, ER) that may be present, and may be inactive. In some cases, inactivation is associated with deletion (i.e. a much shorter domain), but in other cases only a small number of critical residues may be changed. A similarity approach using BLAST (Altschul et al., J. Mol. Biol., 215, 403-410, 1990) suffers from the problems that the weighting of each residue is independent of position and that insertion or deletion of residues is not handled well. In contrast, the hidden Markov model (HMM) approach used in the HMMER suite of programs (Eddy, Bioinformatics, 14, 755-763, 1998), deals with each amino acid position separately and, thus, distinguishes between critical highly conserved residues and less important positions. In addition, the HMMs consider insertion and deletion states and assign them probability. The *hmmalign* program of the *HMMER* suite aligns sequences with respect to the HMM profile allowing accurate identification of particular residue positions. A similar HMM approach was used to predict substrate specificity of NRPS adenylation domains, which has been incorporated into a program package called the *NRPSpredictor* (Rausch et al., Nucleic Acids Res., 33, 5799-5808, 2005; http://www-ab.informatik.uni-tuebingen.de/software/NRPSpredictor/welcome.html).

### Technical solution:

According to this invention, a method was developed to construct a specifically structured customised database of polyketides, non-ribosomal peptides, siderophores, hybrid and other products of modular biosynthetic pathways, as well as a method for: the acquisition, annotation, structuring, storage and graphical presentations of data, establishment of catalytic domain profiles, precise prediction of activities/inactivities and biological functions of modular biosynthetic pathway protein domains, modelling and graphical presentation of biosynthetic processes, modelling and graphical presentation of starter and extender building blocks, modelling and graphical presentation of the *in silico* homologous recombination process, modelling and graphical presentation of the translation of DNA sequences into the linear polyketide, peptide, siderophore, hybrid and other modular biosynthetic pathways chains, as well as modelling and graphical presentation of spontaneous ring formation from the predicted linear products of the recombinant DNA gene-clusters. The described method allows the generation of a library of virtual, entirely novel chemical entities obtained by the *in silico* homologous recombination of modular biosynthetic gene-clusters that can be used for the *in silico* screening for potential biological activity using CDD technology.

The platform independent integral generic program package consists of a novel method for the automatic acquisition, structuring, storage and graphical presentations of data based on *BLAST* searching, coupled with a method for the semi-automatic upload of a specifically structured customised database of polyketide, non-ribosomal peptide, siderophore, hybrid and other products of modular biosynthetic pathways. The specificity of the structured customised database is a comprehensive link between biological and chemical data. The method for importing gene-clusters, genomes or metagenome DNA sequences, either from the Web, hard disk or the server is coupled with an entirely automatic translation into protein reading frames using *BioJava.* The genes are identified using *GeneMark* or *Glimmer,* allowing the identification of PKS, NRPS, NIS, hybrid and other modular gene-cluster protein domains encoded by the genes using the *HMMER* suite of programs constructed from protein profiles in the Pfam database, or entirely customised profiles. The most important and novel solution that none of the existing available programs have achieved is prediction of activity and specificity of each catalytic domain, which is determined using fingerprints based on catalytically important amino acids. The final annotation can be exported as *EMBL, GenBank* or *XML* files for use in other applications, or for submissions to public databases. From the annotated gene-clusters containing predicted activity and specificity of catalytic domains, the module specificities are deduced allowing a prediction of the chemical structures of the linear polyketide/peptide/siderophore/hybrid and other product chains. The structures are represented internally in the program as isomeric SMILES, which can be exported for use with standard chemical software or displayed in the program package using *Jmol.* The program can also produce a putative cyclic structure from the linear molecule. The cyclic aglycon description in SMILES can also be drawn and displayed in the program package using *Jmol* or exported. In the platform independent, integral generic program package a unique modelling and graphical presentation of the *in silico* homologous recombination proccess between genes that encode all the catalytic domains required for the biosynthesis of the cyclic product structure, as well as the generation of a library of virtual novel chemical entities is incorporated. Also, according to this invention it is possible to analyse a given chemical structure to see if it could be produced by PKS/NRPS/NIS/hybrid or other product synthase/synthetase gene-clusters and suggests the DNA sequence of a suitable cluster based on building blocks derived from clusters contained in the custom database. Furthermore, the present invention, enables *in silico* screening of potential therapeutic agents based on CDD technology.

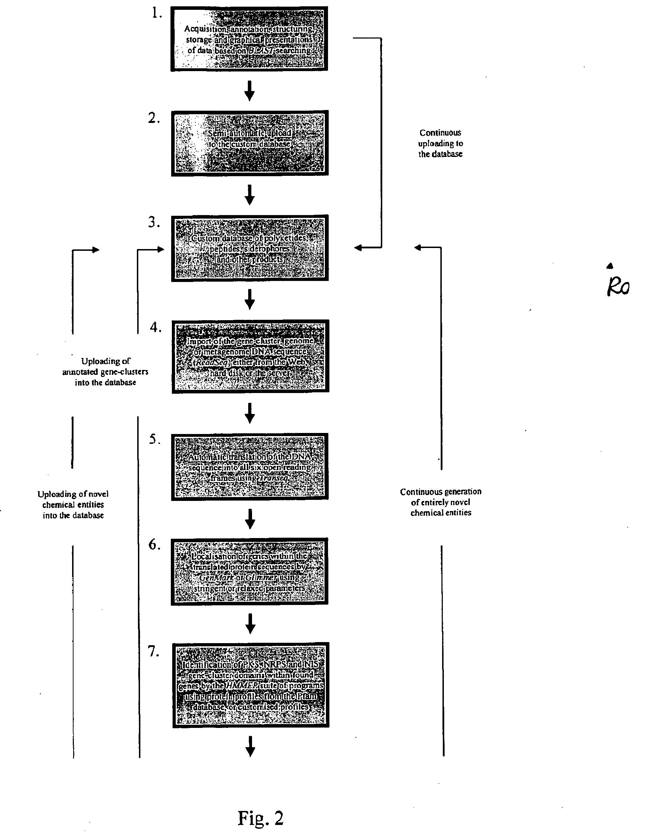

The platform independent, integral generic method was developed using UML (Rumbaugh *et al.,* Addison-Wesley, Boston, 2005). The flow chart of the entire process is given in Fig. 2. The program package supporting the invented methods is written in *Java* and *JavaScript* and runs on a *LINUX* server with a *Java* client on the user's computer compatible with *Windows, MacIntosh* and *Linux* operating systems. It allows the rapid mining of massive DNA data sets to hunt for and to generate novel gene-clusters encoding entirely new compounds that could be the medicines of the future. Most exciting of all, when such a product looks promising *in silico,* a "designer bug" can be created in the laboratory to produce it. Such an analysis of gene-clusters in genomes and metagenomes would have previously taken many weeks and required a user very skilled in the art. A further advantage of the present technical solution over prior art is that with the integrated, generic program package and method disclosed hereinafter, the same tasks can be performed in a matter of hours and requires much less user experience. Therefore, it will increase the productivity of all scientists in industrial organisations and academic institutions, who are faced with extracting data on complex biosynthetic processes from genome and metagenome sequences or other substantial DNA data sets and with the generation of novel chemical entities that can been screened for biological activity and/or used as scaffolds (leads) for further modification by medicinal chemistry.

Also, one of the advantages of the present invention is accuracy. None of the programs available on the market have comparable predictions of enzyme, or enzyme domain activities and specificities.

### Brief description of tables and figures:

Table 1. Specificity of PKS domains
- Fig. 1.: Schematic representation of genes and proteins of the type I multi-modular polyketide synthase responsible for the biosynthesis of erythromycin A. The linear arrangement of genes (*ery*AI, *ery*AII and *ery*AIII) corresponds to the order of proteins (DEBS1, DEBS2 and DEBS3) in biosynthesis. Domains for acyltransferases (AT), acyl carrier proteins (ACP), ß-ketoacyl synthases (KS), ketoreductases (KR), dehydrase (DH), enoyl reductase (ER) and thioesterase (TE) are shown as well as the polyketide aglycon 6-deoxyerythronolide B (6-dEB) and the final product, the macrolide antibiotic erythromycin A. Domains that are involved in β-carbon reduction are shadowed.
- Fig. 2.: The flow chart illustrating the entire process
- Fig. 3.: The flow chart illustrating automatic acquisition, structuring, storage and graphical presentations of polyketide, non-ribosomal peptide, siderophore, hybrid and other products of modular biosynthetic pathways data
- Fig. 4.: The flow chart illustrating semi-automatic upload of the custom database of polyketide, non-ribosomal peptide, siderophore, hybrid and other products of modular biosynthetic pathways data
- Fig. 5.: The structure illustrating relational custom database of polyketides, peptides, siderophores, hybrid and other products
- Fig. 6.: The flow chart illustrating automatic translation of a DNA sequence into all six open reading frames using *BioJava*
- Fig. 7.: The flow chart illustrating localisation of genes within the translated protein sequences by *GenMark* or *Glimmer* using stringent or relaxed parameters.

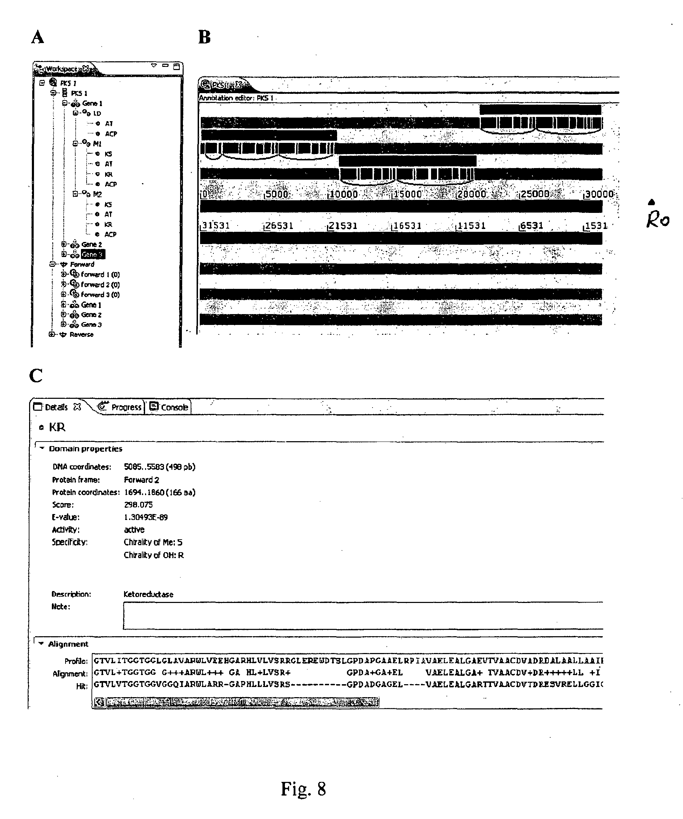

- Fig. 8.: "Annotation editor". **(A)** The "Workspace" window gives an overview of the analysis in the form of collapsible trees. Detected genes and domains are shown. **(B)** The "Annotation editor" window shows the location of genes (in red) and domains (in blue). In this case there are three genes on the three different forward open reading frames. The genes have been displaced from the reading frames by the user to allow better visualisation of the domains. The "Annotation editor" has been used for user definition of modules (shown as red curves below the open reading frames). (C) The "Details" window allows the user to examine the evidence for assignment of domains. The *HMMER* scores and E-values as well as the alignment are displayed. The predictions of activity and specificity are also displayed
- Fig. 9.: "Cluster editor". The user can define a set of contiguous genes as a cluster. The cluster editor window shows the genes in a cartoon form with an expanded view of the selected gene showing domains. Domains can be linked together to give modules. The modules are given identifying names and the program suggests a biosynthetic order that can be accepted or altered by the user
- Fig. 10.: The fingerprints of KR domains
- Fig. 11.: The fingerprints of AT domains
- Fig. 12.: The flow chart illustrating identification of PKS, NRPS, NIS, hybrid and other modular gene-cluster domains within found genes by the *HMMER* suite of programs using protein profiles from the Pfam database, or customised profiles
- Fig. 13.: The flow chart showing prediction of putative chemical structures of the linear polyketide/peptide/siderophore chains and putative cyclic chemical structures and their upload into the custom database
- Fig. 14.: The flow chart illustrating generation of the recombinant DNA sequences, the annotation of recombinant gene-clusters, prediction of putative linear and cyclic products and their upload into the custom database
- Fig. 15.: The flow chart illustrating the optional "reverse genetics" module
- Fig. 16.: The flow chart illustrating prediction of biological activities of the *in silico* generated novel chemical entities using computer tools like PASS and/or CDD technology and their upload into the custom database
- Fig. 17.: Functional prediction of the DNA sequence (AY771999) of the erythromycin synthetic gene-cluster. The three genes (in red), six modules (underlined in red) and 29 catalytically active domains (in light and dark blue) of the erythromycin synthetic gene-cluster are shown **(A).** The isomeric SMILES as well as the 2- and 3-D structure of the predicted linear chain is also shown **(B** top and right). The cyclisation function predicts a ring structure that can also be displayed **(B** left)
- Fig. 18.: Functional prediction of the DNA sequence (AF016585) of the niddamycin gene-cluster. The five genes (in red), seven modules (underlined in red) and 36 catalytically active domains (in light and dark blue) of the niddamycin gene-cluster are shown **(A).** The isomeric SMILES as well as the 2- and 3-D structure of the predicted linear chain is also shown **(B** top and right). The cyclisation function predicts a ring structure that can also be displayed **(B** left)
- Fig. 19.: The annotation of all PKS and NRPS gene-clusters in sequenced genomes of the *Saccharopolyspora erythraea* and *Streptomyces scabies.* The "Workspace" window shows 11 PKS, 6 NRPS and 1 hybrid PKS-NRPS gene-clusters of the *Saccharopolyspora erythraea* genom. The 3 PKS, 4 NRPS and 1 hybrid PKS-NRPS gene-clusters of the *Streptomyces scabies* genome are also shown.

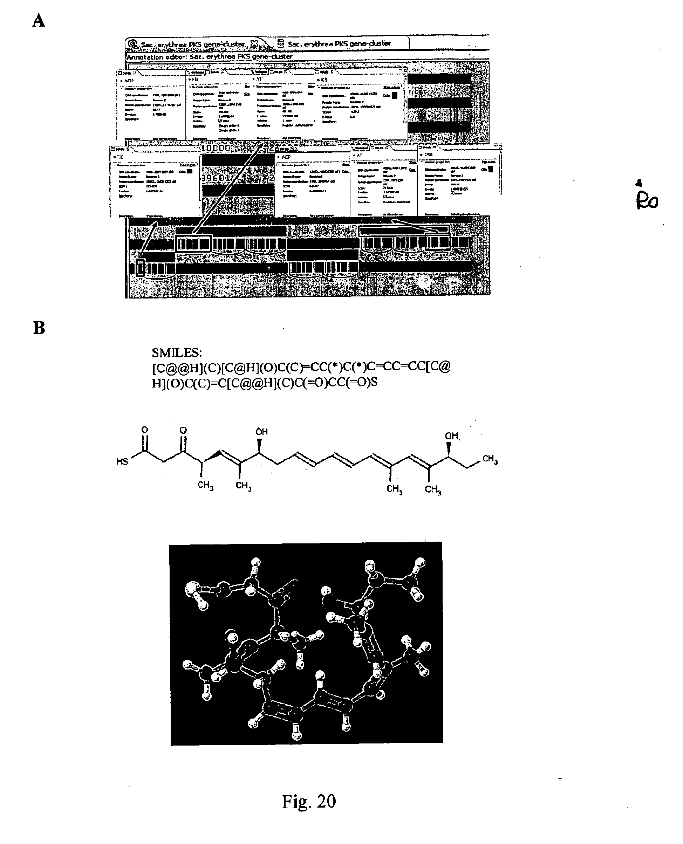

- Fig. 20.: Functional prediction of one gene-cluster from the *Saccharopolyspora erythraea* genome (SACE_4141). The Pke A1-A4 gene-cluster shown contains 8 modules starting with the loading module, housing the initiating decarboxylase, an acyl transferase loading malonyl and the acyl carrier protein domains, and ends with the thioesterase domain. The extender building blocks loaded by the acyl transferase domains and the chyralities of hydroxyl and β-carbon groups by the ketoreductase domains have all been predicted **(A).** An isomeric SMILES as well as the 2- and 3-D structure of the predicted linear octaketide chain are also shown **(B).**
- Fig. 21.: Functional prediction of one putative hybrid modular PKS-NRPS gene-cluster from the Craig Venter's Institute metagenomic dataset. The gene-cluster starts with the NRPS loading module (LD), the adenylation and peptidyl carrier protein domains, followed by three PKS modules and seven NRPS modules, and ends with the NRPS thioesterase domain (Te)
- Fig. 22.: Functional prediction of the DNA sequence of the third recombinant between the 14-membered macrolide erythromycin and the 16-membered macrolide niddamycin gene-clusters. The 3 genes (in red), 4 modules (underlined in red) and 19 catalytically active domains (in dark blue) of the third recombinant gene-cluster are shown **(A).** The isomeric SMILES as well as the 2- and 3-D structure of the predicted linear chain is also shown **(B** top and right). The cyclisation function predicts a ring structure that can also be displayed **(B** left)
- Fig. 23.: Functional prediction of the DNA sequence of the fourth recombinant between the 14-membered macrolide erythromycin and the 16-membered macrolide niddamycin gene-clusters. The 5 genes (in red), 9 modules (underlined in red) and 45 catalytically active domains (in dark blue) of the fourth recombinant gene-cluster are shown **(A).** The isomeric SMILES as well as the 2- and 3-D structure of the predicted linear chain is also shown **(B** top and right). The cyclisation function predicts a ring structure that can also be displayed **(B** left)
- Fig. 24.: Functional prediction of the DNA sequence of the sixth recombinant between the 14-membered macrolide erythromycin and the 16-membered macrolide niddamycin gene-clusters. The 3 genes (in red), 6 modules (underlined in red) and 30 catalytically active domains (in dark blue) of the sixth recombinant gene-cluster are shown **(A).** The isomeric SMILES as well as the 2- and 3-D structure of the predicted linear chain is also shown **(B** top and right). The cyclisation function predicts a ring structure that can also be displayed (B left)

### Detailed Description of the Invention:

The platform-independent integral generic program package consists of a novel method for the automatic acquisition, structuring, storage and graphical presentations of data based on *BLAST* searching, and semi-automatic upload of a specifically structured customised database of polyketide, non-ribosomal peptide, siderophore, hybrid and other products of modular biosynthetic pathways. The specificity of the structured customised database is a comprehensive link between biological and chemical data. The method for importing gene-clusters, genomes or metagenome DNA sequences, either from the Web, hard disk or the server is coupled with an entirely automatic translation into protein reading frames using *BioJava.* The genes are identified using *GeneMark* or *Glimmer,* allowing the identification of PKS, NRPS, NIS, hybrid and other modular gene-cluster protein domains encoded by the genes using the *HMMER* suite of programs constructed from protein profiles in the Pfam database, or entirely customised profiles. The most important and novel solution that none of the existing programs available on the market achieve is prediction of activity and specificity of each catalytic domain, which is determined using fingerprints based on catalytically important amino acids. The final annotation can be exported as *EMBL, GenBank or XML* files for use in other applications, or for submissions to public databases. From the annotated gene-clusters containing predicted activity and specificity of catalytic domains, the module specificities are deduced allowing a prediction of the chemical structures of the linear polyketide/peptide/siderophore/hybrid and other product chains. The structures are represented internally in the program as isomeric SMILES, which can be exported for use with standard chemical software or displayed in the program package using *Jmol.* The program can also produce a putative cyclic structure from the linear molecule. The cyclic aglycon description in SMILES can also be drawn and displayed in the program package using *Jmol* or exported. The platform independent, integral generic program package also consists of a unique method for modelling and graphical presentation of the *in silico* homologous recombination proccess between genes that encode all the catalytic domains required for the biosynthesis of the cyclic product structure, as well as the generation of a library of virtual novel chemical entities. A "Reverse Genetics" module incorporated within the program package analyses a given chemical structure to see if it could be produced by a PKS/NRPS/NIS/hybrid or other product synthases/synthetase gene-clusters and suggests the DNA sequence of a suitable cluster based on building blocks derived from clusters contained in the custom database. The program package also contains the computer program for the *in silico* screening of the potential therapeutic agents based on CDD technology.

Following sections give description of the each step of the method and process disclosed on fig. 2 in more details.

### Acquisition, annotation, structuring, storage and graphical presentations of data based on BLAST searching

Automatic acquisition, structuring, storage and graphical presentations of polyketide, non-ribosomal peptide, siderophore, hybrid and other products of modular biosynthetic pathways data from the publicly available *GenBank* database *(*Wheeler et al., Nucleic Acids Res., 36 Database issue: D13-D21, 2008) using *BLAST* searching (Altschul et al., J. Mol. Biol., 215, 403-410, 1990) was developed. This was done by the use of *BLAST* URL API which allows the access of NCBI's *QBLAST* and searching publicly available database via HTTP-encoded queries. Standard searching parameters, as well as user defined protein sequences of domains, are used through the developed GUI. The results are graphically displayed in a XML format as a table from which the user can choose particular hits and process them further. Chosen hits are then acquired from Entrez Web services via SOAP protocol through their accession numbers. The chosen hits are graphically presented using the "cluster" editor of the program package and annotated further (see: **identification of PKS, NRPS, NIS, hybrid and other modular gene-cluster domains within found genes by the *HMMER* suite of programs using protein profiles from the Pfam database, or customised profiles).** Completely annotated gene-clusters are uploaded and stored in a server located specifically structured relational custom database.

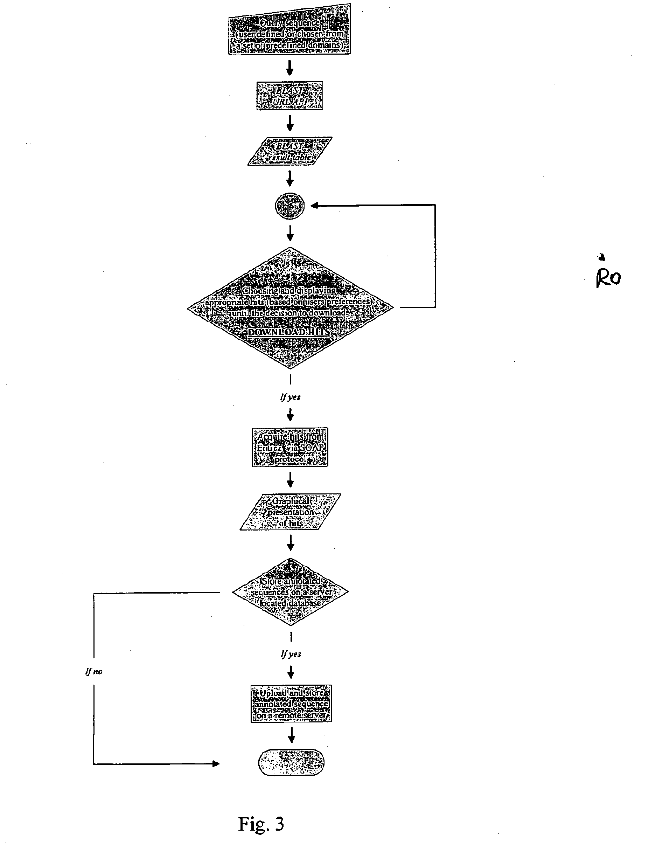

The flow chart illustrating automatic acquisition, structuring, storage and graphical presentations of polyketide, non-ribosomal peptide, siderophore, hybrid and other products of modular biosynthetic pathways data is given in Fig. 3.

### Semi-automatic unload of the custom database

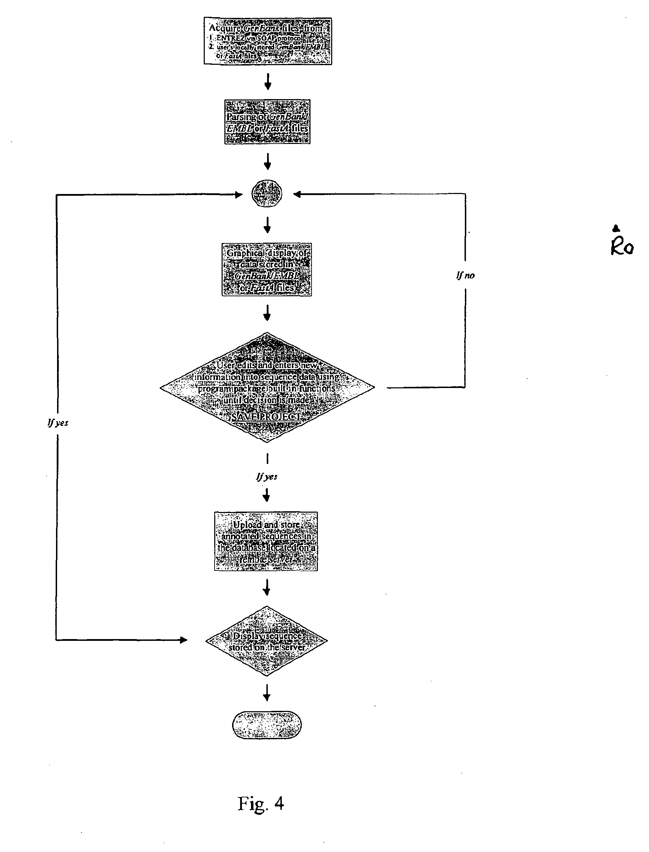

The integral program package has the ability to automatically download *GenBank* files via the accession numbers or BLAST queries, and parse them in order to extract all the information stored within them. This information is conveniently displayed in the "Annotation Editor" for user to edit and enter new annotations or change existing ones. All the information - generic and user added are saved internally into the relational custom database. This is why the process is called semi-automatic. The flow chart of the method for the semi-automatic upload of the custom database of polyketide, non-ribosomal peptide, siderophore, hybrid and other products of modular biosynthetic pathways data is given in the Fig. 4.

**The custom database of polyketides, peptides, siderophores and other products**

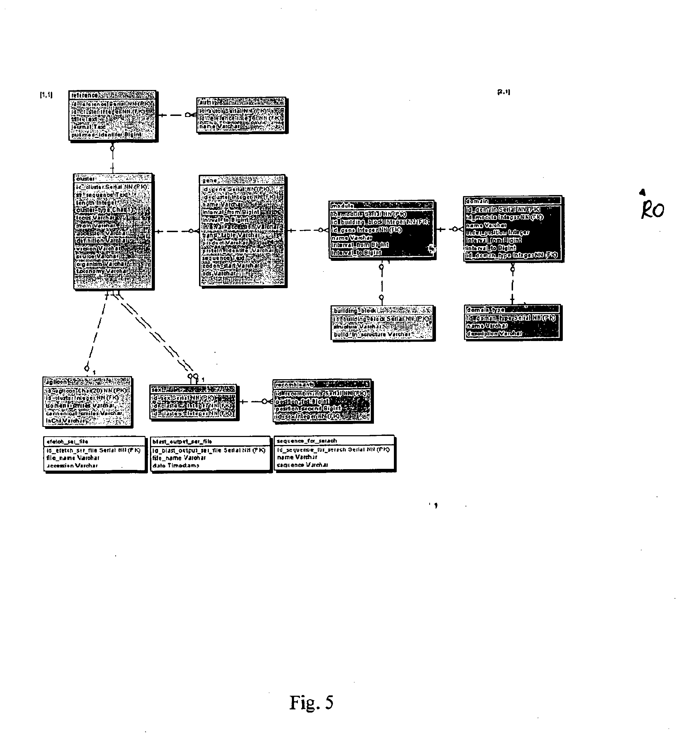

Within the program package there is a specifically structured relational custom database of polyketide, non-ribosomal peptide, siderophore, hybrid and other products of modular biosynthetic pathways data based on BioSQL v 1.29 (Open Bioinformatics Foundation, http://obda.open-bio.org/) as this presents a standard model for representing biological and chemical data. The structure of the custom database is shown in Fig. 5. The specifically structured custom database contain all the data from DNA sequences of well described PKS, NRPS, NIS, hybrid and other modular biosynthetic gene-clusters, as well as clusters that have been deduced on the basis of DNA sequencing of bacterial genome and metagenome data sets (e.g. Bentley et al., Nature, 417, 141-147, 2002; Ikeda et al., Nat. Biotechnol. 21, 526-531, 2003; Oliynyk et al., Nat. Biotechnol., 25, 447-453, 2007, Rusch et al., PLoS Biol. 5, e77, 2007 and others).

The use of clusters derived from sequencing projects is dependent on being able to deduce the function of individual modules. This uses an approach based on hidden Markov models (HMM) and the construction of profiles. A suite of programs was developed for this purpose (see: **Identification of PKS, NRPS, NIS, hybrid and other modular gene-cluster domains within found genes by the *HMMER* suite of programs using protein profiles from the Pfam database, or customised profiles).** Initially the custom database was constructed for PKS gene-clusters, because they are the best known. However, the software was developed around a generic modular model so that it can be applied to NRPS, NIS, hybrid and other modular gene-clusters. A unique feature of the custom database is a description of the biosynthetic process that allows programs to automatically deduce the altered products produced by homologous recombination (see: **Generation of the recombinant DNA sequences, the annotation of recombinant gene-clusters, prediction of putative linear and cyclic products and their unload into the custom database).** This description can also be used to deduce the products resulting from other types of manipulation of the clusters. The custom database also contains the primary protein sequences of all genes, modules, domains and linkers, chemical structures of starter and extender building blocks in isomeric SMILES (Simplified Molecular Input Line Entry System; http://www.daylight.com/smiles/f smiles. html), 2D and 3D structures of polyketide, peptide, siderophore, hybrid and other linear chains to the final chemical structures of polyketide, peptide, siderophore, hybrid and other aglycons. The database contain not only the linear and cyclic structures of the know compounds but also the predicted linear and cyclic structures of the recombinants generated *by in silico* homologous recombination (see: **Generation of the recombinant DNA sequences, the annotation of recombinant gene-clusters, prediction of putative linear and cyclic products and their unload into the custom database).** The database has a suitable interface that allows the user to set queries and to connect all the data stored within the database with the option to extract the data of interest. The specificity of the structured relational custom database is the comprehensive link of biological and chemical data.

### Importing of the gene-cluster, genome or metagenome DNA sequence (ReadSeq) either from the Web, hard disk or the server

The analyses of the sequence data are carried out on a server and the results are cached so that each analysis only needs to be carried out once. This is important as the *HMMER* analysis (see: **identification of PKS, NRPS, NIS, hybrid and other modular gene-cluster domains within found genes by the *HMMER* suite of programs using protein profiles from the Pfam database, or customised profiles)** of a whole *Streptomyces* genome may take several hours. The user accesses the results using a *Java* client that gives user-friendly presentation of the data. There is a password-protected "Workspace" for each user on the server. The client allows the user to upload sequences from any file supported by *ReadSeq* (http://www.ebi.ac. uk/cpi-bin/readseq.cgi), either from the Web, our server or user's hard disk.

### Automatic translation of the DNA sequence into all six open reading frames using BioJava

After import, the DNA sequence is automatically translated into protein reading frames using *BioJava.*

The *BioJava* translates nucleic acid sequences to the corresponding peptide sequence. It can translate in any of the three (3) forward or three (3) reverse sense frames, or in all three forward or reverse frames, or in all six (6) frames. It can translate specified regions corresponding to the coding regions of sequences. It can translate using the standard ('Bacterial') genetic code and also with a selection of non-standard codes. Termination (STOP) codons are translated as the character '*'. The output peptide sequence is always in the standard one-letter IUPAC code.

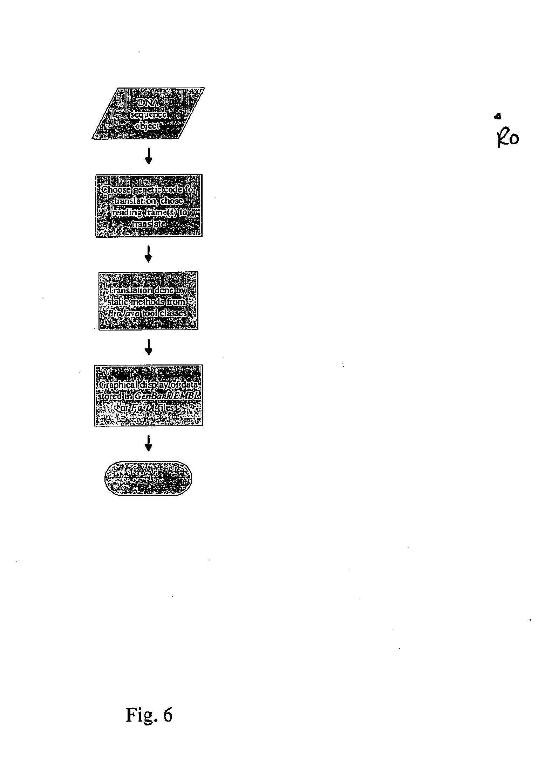

The flow chart of the computer program for the automatic translation of the DNA sequence into all six open reading frames using *BioJava* is given in the Fig. 6.

### Localisation of genes within the translated protein sequences by GenMark or Glimmer using stringent or relaxed parameters

*GeneMark* (version 2.5; http://opal.biology.gatech.edu/GeneMark/; Besemer and Borodovsky, Nucleic Acids Res., 33, Web Server issue W451-454, 2005) or *Glimmer* (version 3.02; http://www.cbcb.umd.edu/software/glimmer/; Delcher et al., Bioinformatics, 23, 673-679, 2007) were used to identify genes. While *GeneMark* offers a fixed list of heuristic models, in the case of *Glimmer* the user can build custom probability models and the use of stringent or relaxed search options are possible. The relaxed search option uses the input sequences to build a Markov model and is, thus, less accurate for shorter sequences or for sequences of high-G+C-content, which have long non-coding open reading frames that can be mistaken for coding regions. For the use of the stringent option *Glimmer* needs a model based on training data of *bona fide* protein coding regions appropriate to the organism being analysed. The user must provide training data in a *FASTA* format, which are used to build the model, which is stored in the custom database. In the case of stringent parameters, the search is more accurate. As models from related organisms work well, it is only necessary to build a limited number of models to cover a wide range of organisms. Putative coding sequences identified by either of these approaches are then graphically displayed in the "Annotation editor". They are shown in the input DNA sequence as s real ratio based on their length (in bp) compared to the length of the entire input DNA (in bp).

The client allows the user to initiate the analyses to find probable coding regions (using *GeneMark* or *Glimmer). GeneMark* uses pre-calculated models for gene finding. The user has to choose the appropriate species from a list. *Glimmer* on the other hand can construct a model for coding regions using long open reading frames (ORF) in the input sequence as training data. This is less effective for short input sequences. Sequences with high G+C-content have long non-coding random ORFs, which may reduce the accuracy of coding sequence prediction. The program, therefore, also allows selection of pre-computed models for particular organism groups. The user can also design models model to be used for gene finding providing the DNA sequences long enough for *Glimmers* "long- ORFs" module.

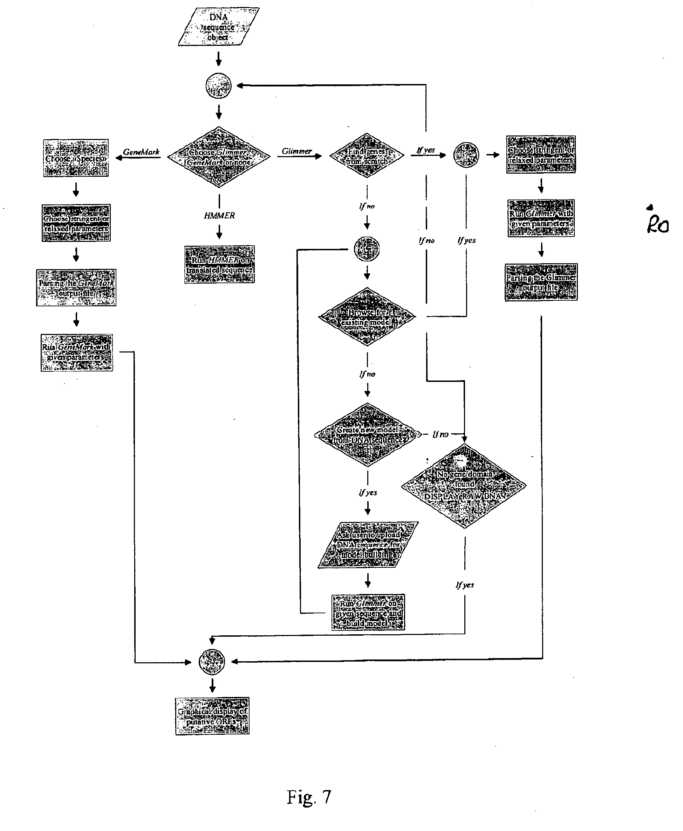

The flow chart of the computer program for the localisation of genes within the translated protein sequences by *GenMark* or *Glimmer* using stringent or relaxed parameters is given in the Fig. 7.

### Identification of PKS, NRPS, NIS, hybrid and other modular gene-cluster domains within found genes by the HMMER suite of programs using protein profiles from the Pfam database, or customised profiles

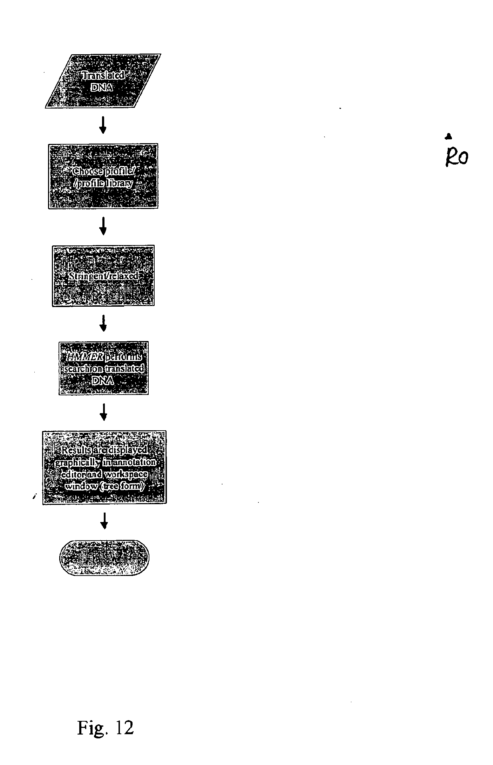

The flow chart for the identification of PKS, NRPS, NIS, hybrid and other modular gene-cluster domains within the found genes by the *HMMER* suite of programs using protein profiles from the Pfam database or the entirely customised profiles is given in the Fig. 12.

The translated DNA sequence was subjected to *HMMER* searches using a library of profiles. The standard libraries contain PKS, NRPS, NIS, hybrid and other product domains, but it is possible to add other libraries if desired. The *HMMER* (version 2.3.2; http://hmmer.janelia.org/; Eddy, Bioinformatics, 14, 755-763, 1998) was used for the identification of domains. Profiles from Pfam (Bateman et al., Nucleic Acids Res., 30, 276-280, 2002) as well as specially constructed profiles were used. For easy and intuitive management of profile data the custom *"HMMER* wrapper" was created and incorporated. The user can search existing libraries of local and global profiles using keywords. The new profiles can be constructed and added. To create a new profile, multiple alignments of training sequences must be provided by either creating multiple alignments from sequences in *FASTA* format or by the insertion of previously generated alignment from *ClustalW.* Profiles as well as alignments can be edited, copied and saved. In other words, they can be handled like any other text file by the computer while the application takes care of their compatibility and warns if exceptions occur. Additionally, profiles can be updated to become more sensitive for new homology searches. At the end, single profile or a list of chosen profiles can be added to the search list. The search can be done using stringent or relaxed parameters. Stringent parameters will recognise any domain identified by the *HMMER* search with an E-value lower than 10⁻³ and the score higher than 0. The relaxed parameters will recognise any domain with an E-value lower than 10 without taking the score into consideration. This covers most possible cases of biologically relevant scores since hits with the E-value higher than 10 are mostly artificial hits. Further filtering of recognised domain hits is provided from the "Annotation Editor" and is done after the *HMMER* finishes its search. The user can filter results based on the score, E-value or combined. The undo and redo options are available to make sure that no relevant result is lost due to filtering.

Raw scores have little meaning without detailed knowledge of the scoring system used, or more simply its statistical parameters *K* and *λ*. Unless the scoring system is understood, citing a raw score alone is like citing a distance without specifying feet, meters, or light years. By normalizing a raw score using the formula S' = λS - K/ln 2 one attains a "bit score" *S*', which has a standard set of units. The *E*-value corresponding to a given bit score is simply E = mn 2^{- s'}. Bit scores subsume the statistical essence of the scoring system employed, so that to calculate significance one needs to know in addition only the size of the search space.

The results are presented in two ways. The "Workspace" window (Fig. 8A) shows the results in a tree format in which branches can be opened up or collapsed. This shows the genes and the domains and is useful for obtaining an overview. More detailed analysis uses the graphical "Annotation Editor" (Fig. 8B) in which the positions of genes and domains are shown on the 6 reading frames - it is possible using the mouse to displace genes and domains above and below the reading frames for better visualisation of overlapping regions. The evidence for the identification of the domains can be viewed using the "Details" window (Fig 8C). This shows the coordinates of the domain in the DNA and protein and the scores and E-values from the *HMMER* analysis. In addition, the alignment of the protein with the profile is shown. On the basis of these details, the user can choose to reject the domain assignment so that it is removed from the display. It is also possible for the user to edit the prediction on the basis of additional knowledge or interpretation of the data in the "Details" window. A history function allows restoration of rejected domains if later desired. The "Details" window also contains predictions of whether the domain is functional and its specificity (if relevant).

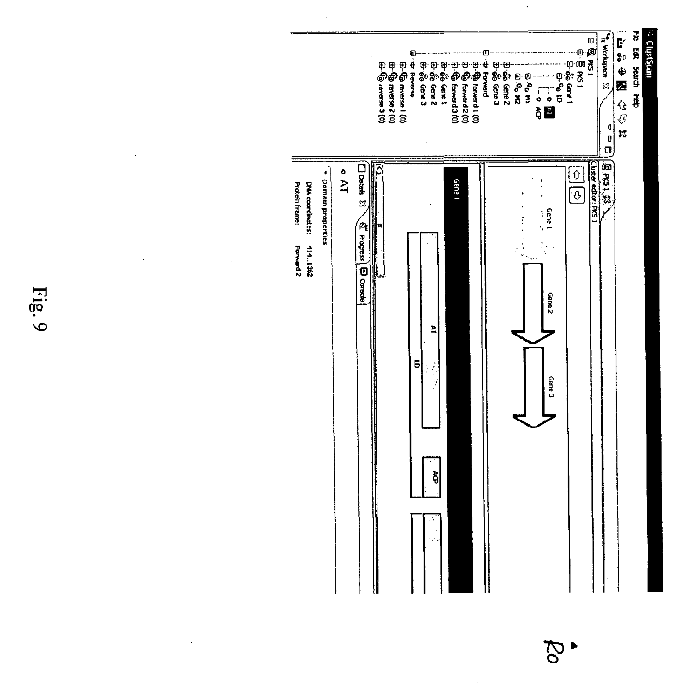

On the basis of the results in the "Annotation Editor", the user can define a cluster covering a region of adjacent genes. The annotation of the cluster is carried out using the "Cluster Editor" (Fig. 9), which shows the genes of the cluster in a simple cartoon form. When a gene is selected, the domains are shown. Modules can be assembled by marking domains and each module created is given a name. The program suggests a biosynthetic order of the genes of a cluster. For PKS clusters this is based on identifying a potential loading domain (i.e. typically a module containing only AT and ACP domains; see: Fig. 9) and looking for a TE domain to identifying the last module. If there is ambiguity, it is assumed that the genes are used in the pathway in the same order as they occur in the DNA. This procedure identifies the correct biosynthetic order in most natural clusters. The user can alter the suggested order to incorporate any additional knowledge available.

The presence of any of the seven domains KS, AT, ER, DH, KR, ACP or TE is detected using the *HMMER* profiles. An extender module needs at least KS, AT and ACP. There is no strong evidence of KS or ACP determining the specificity of a module's activity. In contrast, AT determines the substrate selection for the extension reaction. The three reduction domains (KR, DH and ER) may be absent or present as active or inactive domains. The integral generic program package predicts whether domains are active as well as predicting specificity when several outcomes are possible.

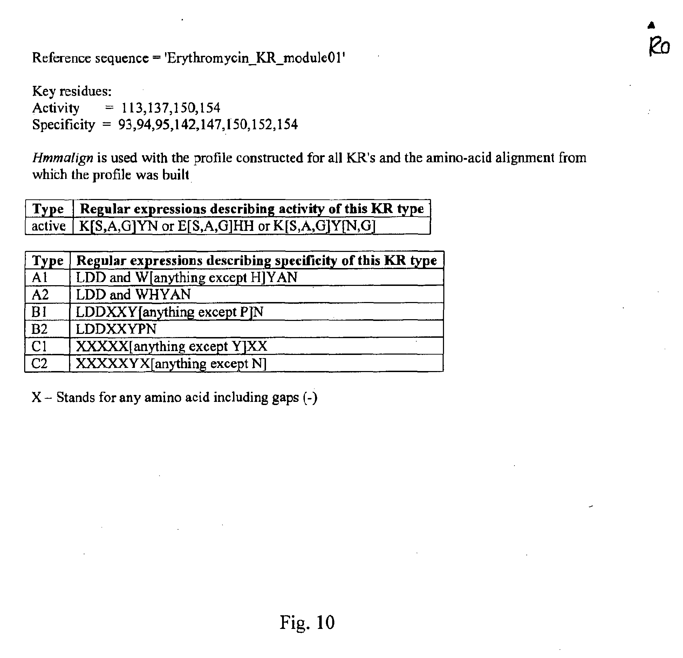

The KR domain is the best characterised domain in terms of structural determination of differential activities. Active KR domains determine the chirality of the hydroxyl product and bioinformatic analysis identified amino acid residues involved in this choice. Bioinformatics also suggested that KR rather than KS determined the stereochemistry of β-carbon groups, when methylmalonyl or ethylmalonyl units are incorporated (see: **Background of the Invention (Prior art)).** As an example, the details of the KR domain fingerprints used are shown in Fig. 10. The fingerprints consist of the important amino acid residues identified from existing knowledge and custom analysis and are compiled in a set of 4 critical residues for the KR activity (K[S or A or G]YN or E[S or A or G]HH or K[S or A or G]Y[N or G]). A set of 8 residues (see below) important for the domain specificity (reduction of α- carbon and the stereochemistry of both α- and β-carbons) were also identified. These residues were mapped and numbered according to the erythromycin KR from the first module of DEBSI in context of a multiple alignment of all polyketide KR domains with known activities and specificities. From this multiple alignment and the two sets of key residues, all possible combinations of active/inactive, as well as different combinations of specificity at the two chiral centres of the KR domain substrates, were assembled in groups. When aligned to a trusted set, all KR's which possess combinations of residues as shown in Fig. 10 under "active" are confirmed as active. Otherwise they are confirmed inactive. For specificity there are six possible combinations because of the two possible chiral centers and in regards to activity or inactivity of the domain, since it was found that the inactive KR domains can also contribute to the specificity of β-carbon group chiral centre (Starcevic et al., ChemBioChem, 8, 28-31, 2007; Keatinge-Clay, Chemistry & Biology, 14, 898-908, 2007). All KR domains having the correct combinations of residues when aligned to the trusted set of KR's are grouped as A1 (LDD and W[anything except H]YAN), A2 (LDD and WHYAN), B1 (LDDXXY[anything except P]N), B2 (LDDXXYPN), C1 (XXXXX[anything except Y]XX) or C2 (XXXXXYX[anything except N]) according to the scheme defined by Keating-Clay (Keatinge-Clay, Chemistry & Biology, 14, 898-908, 2007).

The accuracy of prediction was tested using 49 KR domains for which the structure of the polyketide product provides information about activity and stereochemistry. However, if further active reduction domains are present, the product does not provide any information about the stereochemistry of the KR step. Ten of the KR domains processed malonyl extender units so that only hydroxyl stereochemistry was relevant: all 10 predictions were correct. Nine of the methylmalonyl-processing KR domains were inactive: in 8 cases the program predicted that the domains were inactive for reduction and also predicted the correct β-carbon stereochemistry. In one case the inactive KR domain was predicted as active. The other 30 methylmalonyl-processing KR domains were active. In 25 cases the program predicted the correct stereochemistry. In one case, the program predicted the incorrect methyl stereochemistry (A1 instead of A2). In the other four cases, the alignment with the profile did not yield an amino acid fingerprint that fell into any of the groups: in these cases the program indicates that no prediction is possible. Thus, the KR prediction was correct in 88% of the cases, incorrect in 4% of the cases and the program was unable to provide a prediction in 8% of the cases.

Unlike the case of KR, structural information about AT domains is not sufficient to help in substrate prediction. The most common extender substrates are malonyl-CoA and methylmalonyl-CoA. Comparison by eye of alignments of AT domain sequences identified 13 amino acid residues (QQGHS[L or V or I or F or M]GR[F or P]H[A or N or T or G or E or D or S or -][N or H or Q]V for malonyl-CoA and QQGHS[Q or M or I]GRSHT[N or S]V for methylmalonyl-CoA), which differed significantly between domains incorporating the two substrates (see: **Background of the Invention (Prior art)).** The amino acid sequences of 9 AT extender domains that incorporated ethylmalonyl-CoA were examined. It was found that the 13 amino acid residues had a common pattern (Q[Q or H]G[H or S]S[Q or L]GR[G or T or A]HTNV) that differed from those of the malonyl-CoA and methylmalonyl-CoA-specific AT domains. This information was used for the prediction of specificity in the program. A further known extender substrate is methoxymalonyl-CoA. Eleven methoxymalonyl-incorporating AT domains were examined, but the 13 residues did not show a conserved pattern. However, it was noticed that most of them had insertions with respect to the conserved alignment of all AT domains. It was possible to identify two classes of insertions that could be used to predict methoxymalonyl-CoA as a substrate. The first class has a conserved W while the second has conserved P. Since both W and P are rare amino acids, any AT domain which has either one on the correct position in the alignment is affirmed as methoxymalonyl incorporating AT domain.

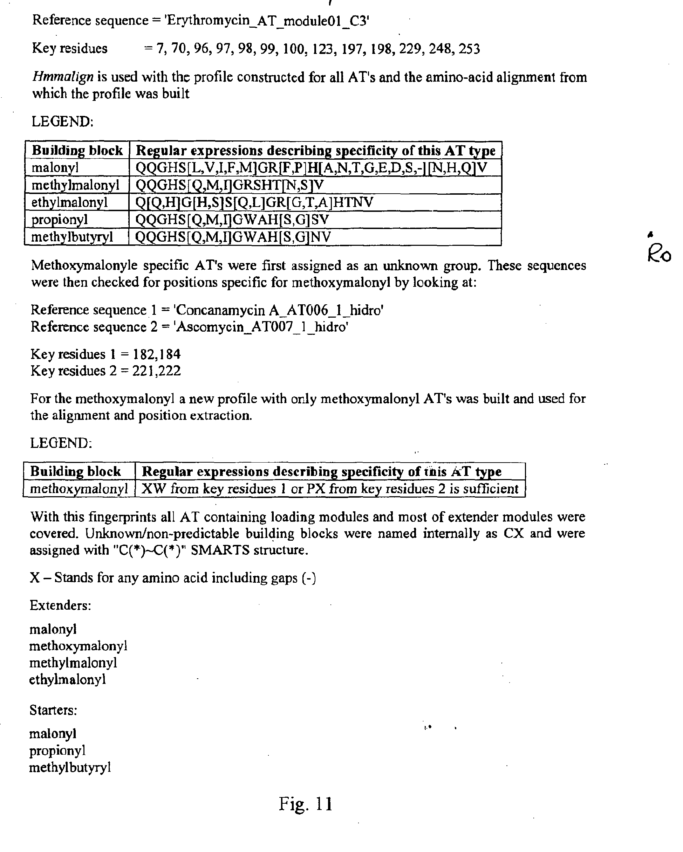

The information about AT extender specificity was implemented in the integral generic program package. The general AT-profile was used to align the AT being analysed to extract the 13 diagnostic amino acid residues. These were compared to three fingerprints corresponding to the three extender substrates (malonyl, methylmalonyl and ethylmalonyl). If the amino acids did not fit any of the three fingerprints, the AT domain was aligned using a profile derived from the 11 methoxymalonyl-CoA AT domains. This alignment was used to test if one of the characterised insertions was present. If no match was found, the AT domain was assigned to an unknown substrate category. As an example the details of the AT domain fingerprints used are shown in Fig. 11. Key amino acid residues were identified from the existing knowledge and custom analysis and are compiled in a set of 13 residues critical for AT specificity. All these residues were mapped and numbered according to the erythromycin AT domain from the first module of the DEBS 1 in context of a multiple alignment of trusted AT domains with known specificities. Additionally, the 2 sets containing characterised insertion of 2 residues important for methoxymalonyl specificity were defined. In the case of methoxymalonyl the specificity was determined according to the concanamycin AT domain from the sixth module for the set 1 and according to the ascomycin AT domain from the seventh module for the set 2. From the multiple alignments of 13 critical residues and the multiple alignments with the two sets of key methoxymalonyl residues the program package correctly predicts building blocks of AT domains as malonyl, methylmalonyl, ethylmalonyl or methoxymalonyl far better than any program available on the market. The unique graphical display of found domains was also developed. In it the user can see the detailed description of the domain specificity and has the ability to change these predictions manually if better information is available. The complete process of prediction is conducted in the two steps. The first step generates the multiple alignment of newly identified AT with the trusted set of AT's. In the second step this alignment is parsed and checked for the key residues in the 2D matrix. The process is completely automated and the user has simple and accurate prediction in the form of "Details" window attached to graphical presentation of the domain in the "Annotation Editor".

The 196 known extender AT domains were analysed with the integral generic program package (95 malonyl-CoA, 79 methylmalonyl-CoA, 9 ethylmalonyl-CoA and 13 methoxymalonyl-CoA. The remaining 25 were propionyl, acetyl, methylbutyryl and some unusual ones from loading module AT's). This gave the correct prediction in 182 cases (93%), the wrong prediction in 9 cases (5%) and assignment to an unknown class in 5 cases (3%).

In addition to AT domains in extender modules, there are often AT domains in starter modules. A set of AT starter domains that incorporate acetyl starters (9 domains), propionyl starters (8 domains) and methylbutyryl starters (3 domains) were aligned with the AT profile and the 13 diagnostic amino acid residues extracted. The acetyl and propionyl starter domains showed the same fingerprint as malonyl-CoA and methylmalonyl-CoA extenders respectively (QQGHS[L or V or I or F or M]GR[F or P]H[A or N or T or G or E or D or S or -][N or H or Q]V for malonyl-CoA and QQGHS[Q or M or I]GRSHT[N or S]V for methylmalonyl-Co). The methylbutyryl starters showed a different pattern (QQGHS[Q or M or I]GWAH[S or G]NV), which was also used to construct a specific fingerprint. This information was incorporated into the integral generic program package. When the user accepts the suggested biosynthetic order, or defines a different order, the starter module is subjected to special analysis. This special analysis requires user intervention. The user must decide which module is a loading module and label it as such. The program then automatically assigns new starter building block prediction by the custom substitution scheme. The custom substitution scheme predicts that when the malonyl-CoA AT's are present within the loading module, the loading module AT will incorporate acetate. On the other hand, when the methylmalonyl-CoA AT's are present within the loading module the loading module AT will incorporate propionate. The propionate will also be incorporated when the specific fingerprint is present (QQGHS[Q or M or I]GWAH[S or G]SV). If an AT domain is present, the 13 diagnostic amino acids are extracted and tested for acetyl, propionyl or methylbutyryl fingerprints. For the 21 AT domains tested, 18 (86%) were correctly predicted and all the others were assigned to the unknown class (14%).

For DH domains, the prediction should firstly distinguish active and inactive domains. As insufficient structural information was available to make predictions based on the knowledge of function, it was decided to use a profile based on active domains to try to predict activity. The profile was built using the sequences of 57 active domains derived from actinomycetes. This custom profile was used to screen the active domains used in its construction as well as new 56 active and 46 inactive domains (159 in total). All domains with a high score (>300) were active, whereas all with a low score (< 200) were inactive. About 80% of the domains with intermediate scores were active, but the scores of inactive domains were distributed throughout the range. These results were used to define a prediction function with three outcomes: active (score > 300), 80% probability of activity (scores between 200 and 300) and inactive (score < 200).

This prediction function was tested on 159 domains (113 active and 46 inactive). Forty six domains fell into the intermediate region (36 active, 10 inactive) with a prediction of 80% probability of activity. Sixty seven domains were predicted to be active of which 6 were in fact inactive corresponding to a 9% false prediction rate. In contrast, the prediction of inactivity was less satisfactory: 43 DH domains were predicted to be inactive of which 16 were actually active. A closer examination of these false predictions showed that only one of the 16 was an actinomycete sequence, the other 15 being sequences from Gram-negative bacteria. When attention was confined to actinomycete sequences, 13 DH domains were predicted to be inactive of which only one was active. The DH domains are involved in the *cis*/*trans* orientation at the double bond. This orientation was also predicted by fingerprints of specific aminoacid residues within domains.

Initially, a similar approach to that used for the DH domain was attempted with the ER domains. A profile was constructed using active actinomycete ER domains. However, it was found that better prediction was achieved with a profile based on a mixture of active and inactive domains. Sixty six known ER domains were tested. In all cases the ER domain was detected. The *HMMER* score did not prove useful in distinguishing between active and inactive ER domains. However, there were only 3 cases of inactive ER domains in the presence of an active DH domain. There were 4 cases in which an ER domain was detected, but the DH domain was inactive. The program, therefore, predicts an active ER domain if a domain is found and there are active KR and DH domains present. This gives a false prediction in 3/66 (5%) cases of an inactive ER domain with an active DH domain. The ER domains are involved in the stereochemistry of α- and β-carbons if the double bond is formed. This stereochemistry was also predicted. None of the programs available on the market have comparable predictions of enzyme domain functions and specificities.

### Export of final annotation as EMBL, GenBank or XML files for use in other applications, or for submissions to custom or public databases

The final annotation can be exported as *EMBL, GenBank* or *XML* files for use in other applications, or for submissions to custom database or to public databases. In addition, the DNA or amino acid sequences of genes, domains or modules can be copied to the clip board for further analyses.

### Prediction of putative chemical structures of the linear polyketide/peptide siderophore/hybrid and other product chains and their upload into the custom database

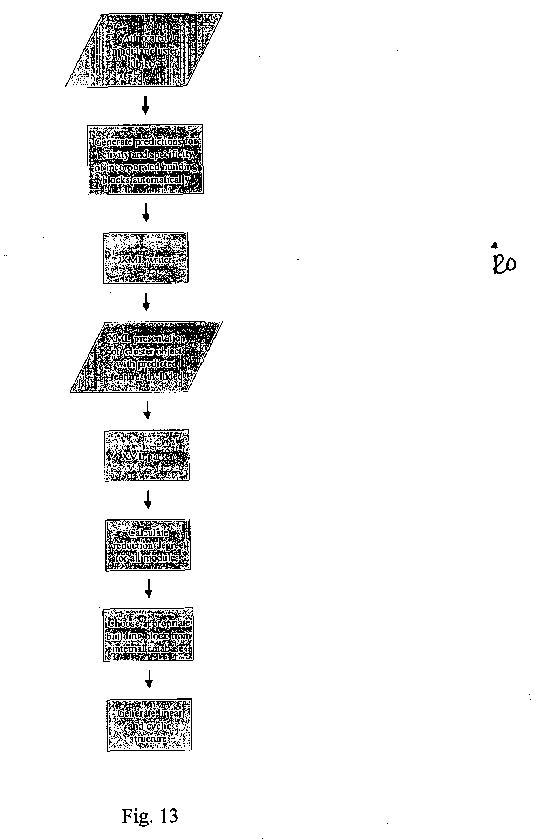

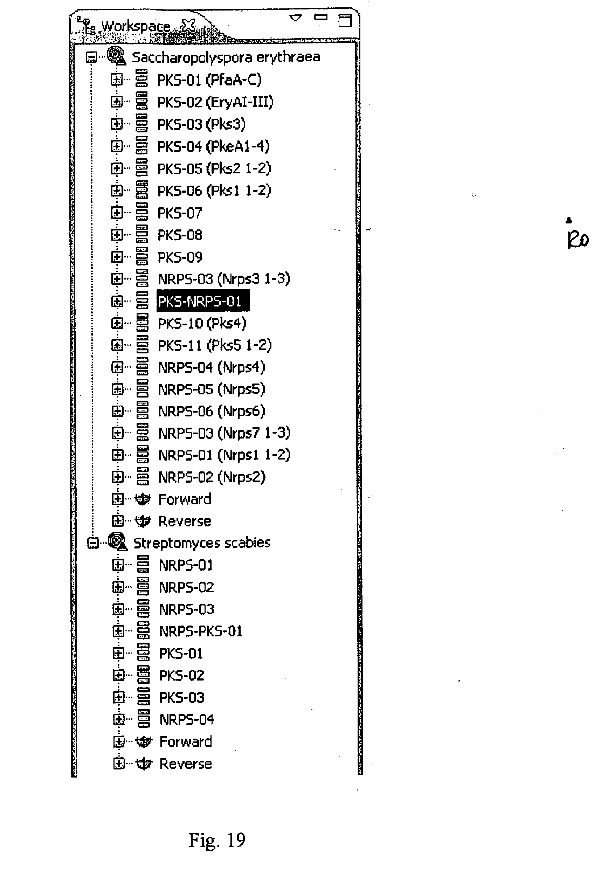

The flow chart for the prediction of putative chemical structures of the linear polyketide/peptide/siderophore/hybrid and other product chains and their upload into the custom database is given in the Fig. 13. The prediction functions for the activity and specificity of domains are used to deduce module specificity and, thus, to predict the chemical structure of the linear polyketide/peptide/siderophore/hybrid and other product chains of gene-clusters. The structures are saved within the program as the isomeric SMILES (Simplified Molecular Input Line Entry System; http://www.daylight.com/smiles/f_smiles. html), which can be exported for the use with standard chemical software. In addition, the program shows molecules in a 3-D "Molecules" window (see. Fig. 19A of the **Example 1)** in which the molecule can be rotated. The linear chain description in SMILES can be drawn and displayed in the program package using *Jmol* v. 11.2.14, 2006 (Jmol; http://www.jmol.org/) or exported.

The present invention models the translation of PKS/NRPS/ /NIS/hybrid and other modular biosynthetic pathways DNA sequences into the linear polyketide/peptide/siderophore /hybrid and other modular biosynthetic pathways chains by the use of a custom method. In this method, the known extender substrates (building blocks) have been organized into the custom database according to their type, reduction degree, stereochemistry and isomerisation. When the user recognise the module and marks it in the protein sequence as such, using the "Annotation editor", the program package automatically predicts the building block. The program deduces building block reduction degree based on the domain composition within the module and predicts its stereochemistry and isomerisation. After fulfilling all these steps, the correct building block is chosen from the custom database. If for some reason there is no prediction for the reduction degree, stereochemistry or isomerisation, or the user wants to discard it, the program package still incorporates building block in which the data for the reduction degree, stereochemistry or isomerisation are left blank. Such "blank" versions of building blocks where the user can manually "fill in the gaps" are also in the custom database and are linked to the appropriate building block.

Most of the linear chemical structures produced are expected to rearrange spontaneously to produce ring structures. Different approaches have been used to model this process.

### Prediction of putative cyclic chemical structures from the linear polyketide/peptide/siderophore/hybrid and other product chains and their upload into the custom database

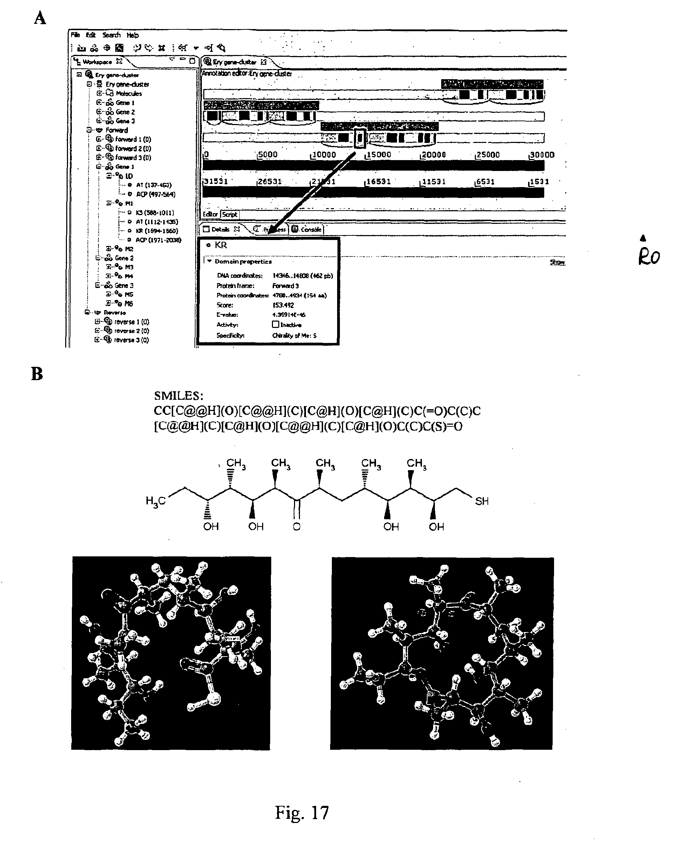

The present invention produces a potential cyclic structure from a linear molecule using several options. The simplest option assumes that the first hydroxyl or amino group introduced during synthesis reacts with the terminal extender unit (see: Fig. 19B of the **Example 1).** A more sophisticated cyclisation model was also implemented with prediction of spontaneous ring formation from the predicted linear products of the recombinant clusters. Two approaches were used. In the first approach, when molecules are small enough, they are accessible to quantum mechanical calculations. A second approach was a program based on chemical knowledge by the use of Gaussian 03 or AMBER 8 suite of programs (Frisch et al., Gaussian 03, Revision C.02, 2004; Case et. al., J. Computat. Chem. 26, 1668-1688, 2005). The cyclic aglycon description in the isomeric SMILES can be drawn and displayed in the program package using *Jmol* v. 11.2.14, 2006 (Jmol; http://www.jmol. org/) or exported. After the linear product has been predicted, all possible cyclisation points are selected. The classical molecular dynamics simulation is firstly performed for each of these potential structures to get a viable view of active site TE domain-linear chain complex. Then the potential of the mean force is calculated to assess feasibility of each cyclic structure. Structures are then sorted depending to how feasible they are. The most feasible is chosen and presented as SMILES or in the graphical form (see: Fig. **17B** top and left).

The flow chart for the prediction of putative cyclic chemical structures from the linear polyketide/peptide/siderophore/hybrid and other product chains and their upload into the custom database is also given in the Fig. 13.

### Generation of the recombinant DNA sequences, the annotation of recombinant gene-clusters, prediction of putative linear and cyclic products and their upload into the custom database

The present invention provides modelling and graphical presentation of *in silico* homologous recombination proccess by the use of *Java* to allow portability and exchange of software components. The extensive *BioJava* library was used for standard sequence handling. The extensive *BioJava* library was used for standard sequence handling The main initial task was to design a Cluster class, which is the main object for handling clusters and modelling recombination. The Cluster class correspond closely to the cluster entries in the custom database. In particular, it contains both sequence information and information about module functionality so that the linear synthesis product can be deduced. The Cluster class forms the basis of software development for a variety of purposes. A recombination method was developed that generates recombinant Cluster objects. The model was implemented with the assumption that a certain minimum length of perfect homology, MEPS (typically 27 bp; Shen and Huang, Genetics, 112, 441-457 1986) is needed and that the surrounding sequences will possess a sufficiently high similarity (typically at least 75% similarity in the surrounding 200 bp). This program includes a variety of recombination models and was constructed so that new models can easily be incorporated. This includes identification of potential chi-sites (Chedin et al., Mol. Microbiol., 29, 1369-1377, 1998) by screening the genomic sequences of actinomycetes. They will be used to examine in detail the theory suggested by preliminary results that the module pairs that recombine are fairly insensitive to the exact recombination model used. The recombinant clusters are analysed by further programs to deduce the linear products produced. This is done by using the biosynthetic descriptions of the clusters.

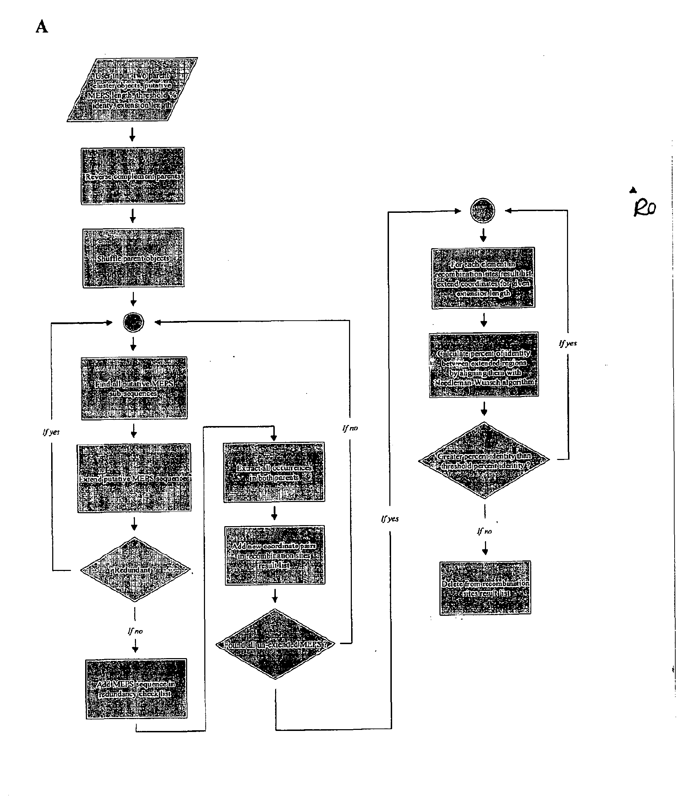

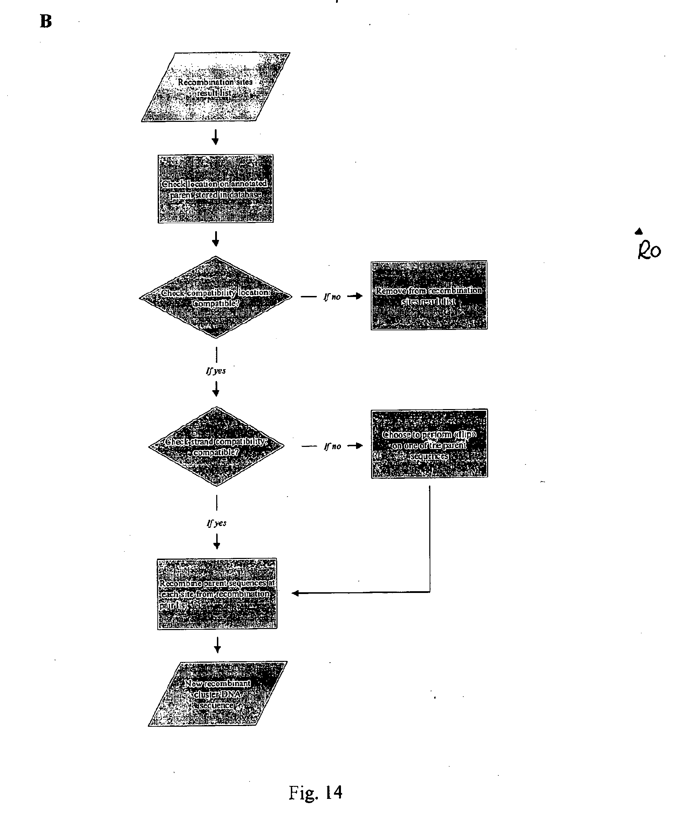

The flow chart of the method for the generation of the recombinant DNA sequences, the annotation of recombinant gene-clusters, prediction of putative linear and cyclic products and their upload into the custom database is given in the Fig. 14.

### "Reverse Genetics" module

A "Reverse Genetics" module analyses a given chemical structure to see if it could be produced by a PKS, NRPS, NIS, hybrid or other product synthes/synthetase gene-cluster and suggests the DNA sequence of a suitable cluster based on building blocks derived from clusters contained in the custom database. The entire process uses a disclosed method and it can be defined as an inversion of the process presented in the fig 2.

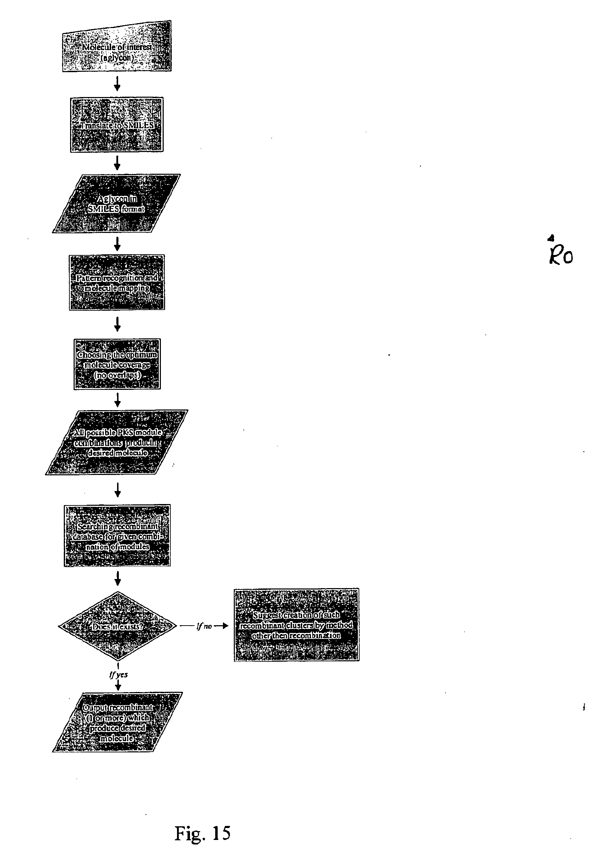

To accomplish this, the given structure must be presented in the isomeric SMILES format. If it is in other common chemical file format, the program will automatically convert it using OpenBabel/JOELib. The resulting SMILES string is then subjected to pattern recognition algorithm analysis. In the simplest case, results of this analysis reveal if there is a recombinant cluster present in the custom database which can produce this molecule. In the case where there is no simple answer, the program package outputs possible module combinations which could produce given molecule from various gene-clusters if combined properly by methods other than homologous recombination. The flow chart illustrating "reverse genetics" module is given in the Fig. 15.

### Prediction of biological activities of the in silico generated novel chemical entities using CDD technology and their upload into the custom database

Furthermore, the present invention *provides in silico* screening of the potential therapeutic agents based on Computer aided drug design technology (CDD; see references in: Zauhar et al. J. Med Chem., 46, 5674-5690, 2003).

Prediction of the biological activities of the novel chemical entities generated by *in silico* recombination and stored in the custom database is done in a top-down manner. Methods for the prediction of biological activity use both ligand and structure based CDD methods. These methods require additional chemical structures of known natural products, commercially available compounds and drugs stored in databases such as "ZINC" (Irwin and Shoichet, J. Chem. Inf. Model., 45, 177-182, 2005) and structures of the target active sites. The whole process then comes down to the filtering of novel chemical entities generated by the *in silico* recombination, which are not recognised as biologically active, so that only the potential leads stay in the database.

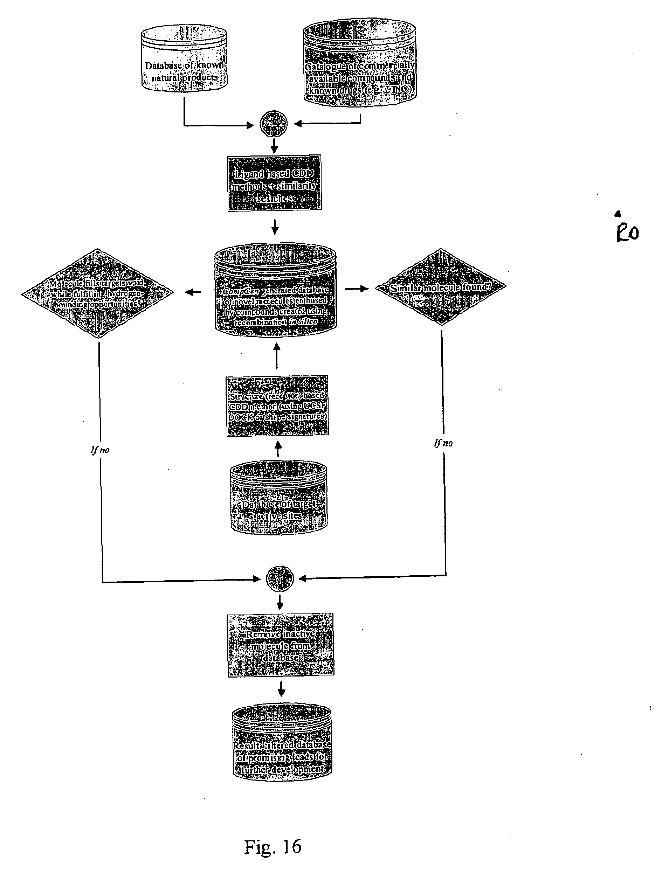

The flow chart of the method for prediction of biological activities of the *in silico* generated novel chemical entities based on CDD technology and their upload into the custom database is given in the Fig. 16.

The present invention is designed to allow easy incorporation of new knowledge. New or modified prediction of activity or specificity can be implemented without changing the program package structure. It is also possible for sophisticated users to write their own specific scripts to introduce specialised prediction functions.

### Example 1

The functional prediction of the DNA sequence of the 14-membered macrolide erythromycin synthetic gene-cluster (AY771999; Kodumal et al., Proc. Natl. Acad. Sci. U S A., 101, 15573-15578, 2004) is shown in Fig. 17A. The integral generic program package correctly predicted three genes (*ery*AI*, ery*AII and *ery*AIII), three proteins (DEBS1, DEBS2 and DEBS3), six modules and 29 catalytically active domains of the erythromycin synthetic gene-cluster. It also predicts that the loading AT domain loads propionyl-CoA while the six AT domains of extender modules loads methylmalonyl-CoA. The correct predictions of the α-and β-carbons stereochemistry by the KR's and ER's were also achieved. In particular, the inactive KR in module 3 responsible for the β-carbon: S stereochemistry was predicted. The linear chain and the cyclic (6-deoxyerythronolide B) aglycon structures were also correctly predicted. The synthetic erythromycin gene-cluster linear and cyclic products are shown in Fig. 17B.

### Example 2

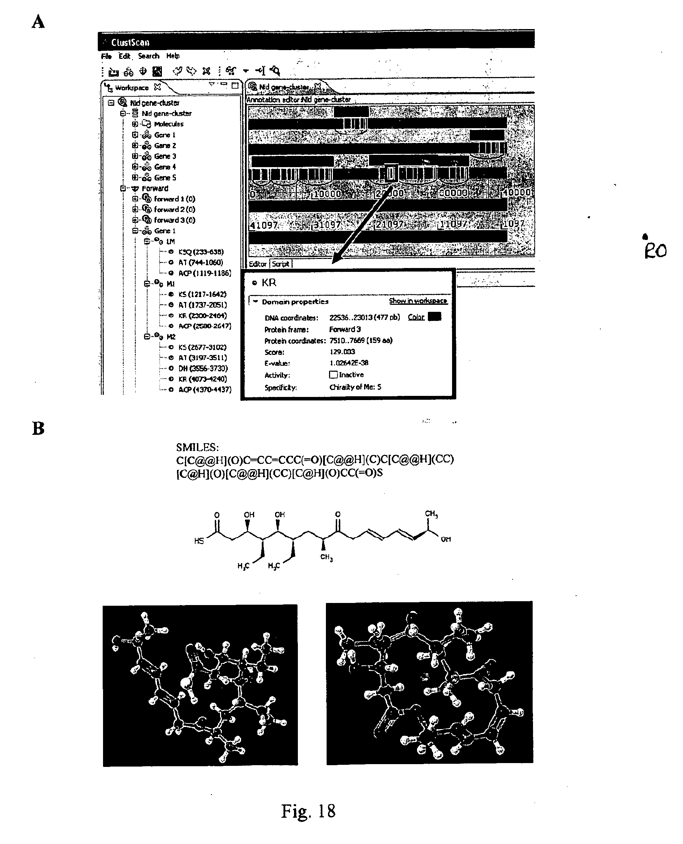

The functional prediction of the DNA sequence of the 16-membered macrolide niddamycin gene-cluster (AF016585; Kakavas et al., J. Bacteriol., 179, 7515-7522, 1997) is shown in Fig. 18A. The integral generic program package correctly predicted five genes (*nid*AI, *nid*AII, *nid*AIII, *nid*AIV and *nid*AV), five proteins, seven modules and 36 catalytically active domains of the niddamycin gene-cluster. It also predicts that the loading AT domain loads, after the initial decarboxylation by the KS^{Q}, acetyl-CoA while the other seven AT domains of the extender modules load four malonyl-CoA, one hydroxymalonyl-CoA, methylmalonyl-CoA and ethylmalonyl-CoA, respectively. The correct predictions of the α-carbon reduction and the α- and β-carbon stereochemistry by the KR's and ER's were also achieved. In particular, the inactive KR in module 4 responsible for the β-carbon: S stereochemistry was predicted. The linear chain and the cyclic aglycon structures were also correctly predicted. The niddamycin gene-cluster linear and cyclic products are shown in Fig. 18B.

### Example 3

During the last year Peter Leadlay's group, at the University of Cambridge, published the DNA sequence of the *Saccharopolyspora erythraea* genome, the producer of erythromycin (Oliynyk et al., Nat. Biotechnol., 25, 447-453, 2007). In addition to erythromycin gene-cluster, they have identified 11 PKS, 6 NRPS and 1 hybrid PKS-NRPS gene-cluster whose products are yet unknown. The *Streptomyces scabies* genome was also sequenced but not yet published. Using the platform independent integral generic program package all PKS and NRPS gene-clusters were annotated in both genomes in about three hours of work (Fig 19). In the *Saccharopolyspora erythraea* genome the program package identified 11 PKS, 6 NRPS and 1 hybrid PKS-NRPS gene-clusters that are identical to those previously described. On the other hand, in the *Streptomyces scabies* genome the program package identified 3 PKS, 4 NRPS and 1 hybrid PKS-NRPS gene-clusters.

If someone is performing the same task with the same tools individually, it will take three to four weeks.

### Example 4

One gene-cluster from the *Saccharopolyspora erythraea* genome (SACE_4141-4138) is shown in Fig 20A as an example. The Pke A1-A4 gene-cluster shown contains 8 modules starting with the loading module, housing the initiating decarboxylase, an acyl transferase selecting malonyl-CoA and the acyl carrier protein domains, and ends with the thioesterase domain. The extender building blocks loaded by the acyl transferase domains and the chiralities of hydroxyl and β-carbon groups by the ketoreductase domains have all been predicted. Therefore, the integral generic program package confirmed Leadly's group annotation and adds the prediction of stereochemistry. The program package also predicts a linear octaketide chain (Fig 20B).

Similar gene-clusters, from two *Streptomyces* species and one myxobacteria, have already been analysed by Ecopia BioSciences Inc. (Banskota at al., J. Antibiot. 59, 168-176, 2006) and by Rolf Müller's group from Saarbrücken (Frank et al., J. Mol. Biol. 374, 24-38, 2007). They have showed similar octaketide structures.

### Example 5

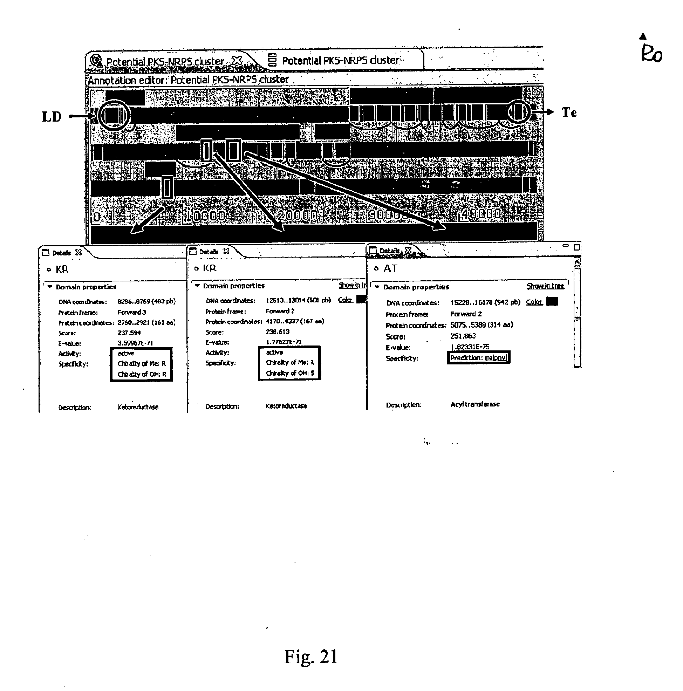

The J Craig Venter Institute ocean metagenomic dataset (Rusch *et al., PLoS Biol. **5***, e77, 2007) was also analysed by the integral generic program package. The functional prediction of one putative hybrid modular PKS-NRPS gene-cluster is shown in Fig 21 as an example. The gene-cluster starts with the NRPS loading module, the adenylation and peptidyl carrier protein domains, followed by three PKS modules and seven NRPS modules, and ends with the NRPS thioesterase domain. At present, the program package can predict the PKS AT and KR domains specificity. Although there is a space in the amino acid sequence, the two AT domains were not detected and they might determine novel chemistry that is novel extender units.

### Example 6

The recombination between the 14-membered macrolide erythromycin and the 16-membered macrolide niddamycin gene-clusters from *Saccharopolyspora erythraea* NRRL23338 and *Streptomyces caelestis* NRRL2821 chromosomes were *performed in silico.* For *recombination in silico,* sequences from the erythromycin synthetic gene-cluster (AY771999) and the niddamycin gene-cluster (AF016585) were used. The recombination generated 8 recombinants from which the 3^{rd}, 4^{th} and 6^{th} were chosen for further analysis.

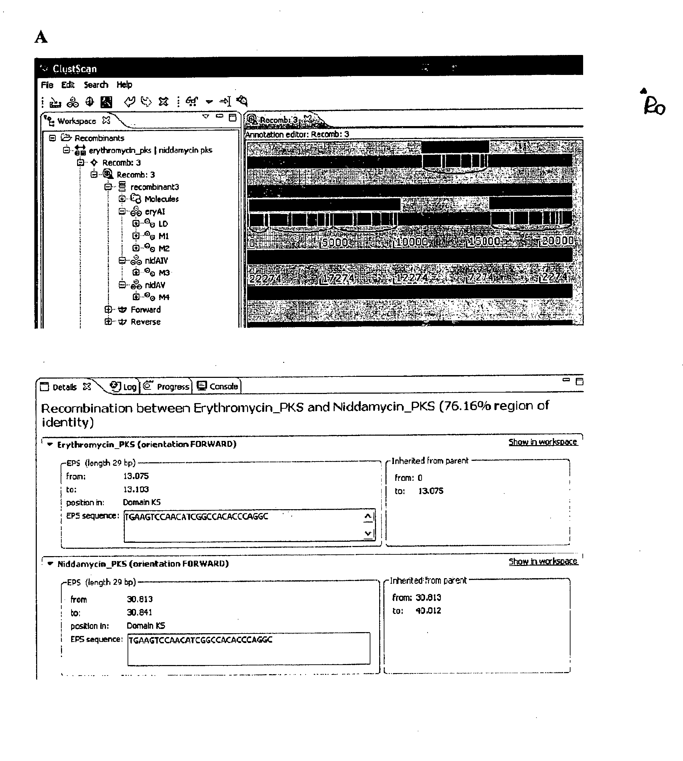

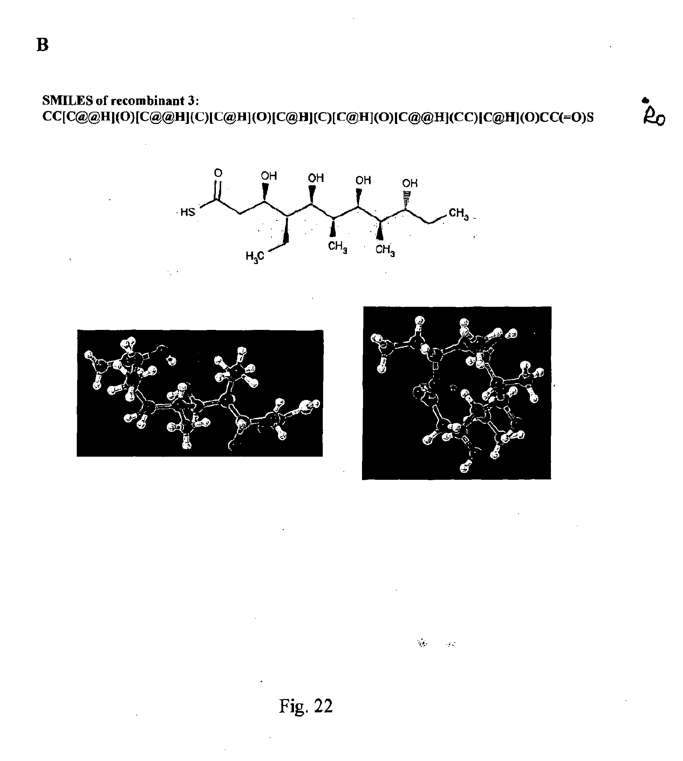

The functional prediction of the DNA sequence of the 3^{rd} recombinant between the 14-membered macrolide erythromycin and the 16-membered macrolide niddamycin gene-clusters is shown in Fig. 22A. The method predicts 3 genes (*ery*AI, *nid*AIV), 3 proteins, 4 modules and 19 catalytically active domains of the third recombinant gene-cluster. It also predicts that the loading AT domain loads propionyl-CoA while the other four AT domains of the extender modules loads one malonyl-CoA, two methylmalonyl-CoA and one ethylmalonyl-CoA, respectively. The correct predictions of the α-carbon reduction and the α- and β-carbons stereochemistry by the KR's and ER's were also achieved. The linear chain of the 12-membered macrolide and its cyclic aglycon structure were also predicted. The third recombinant gene-cluster linear and cyclic products are shown in Fig. 22B.

### Example 7

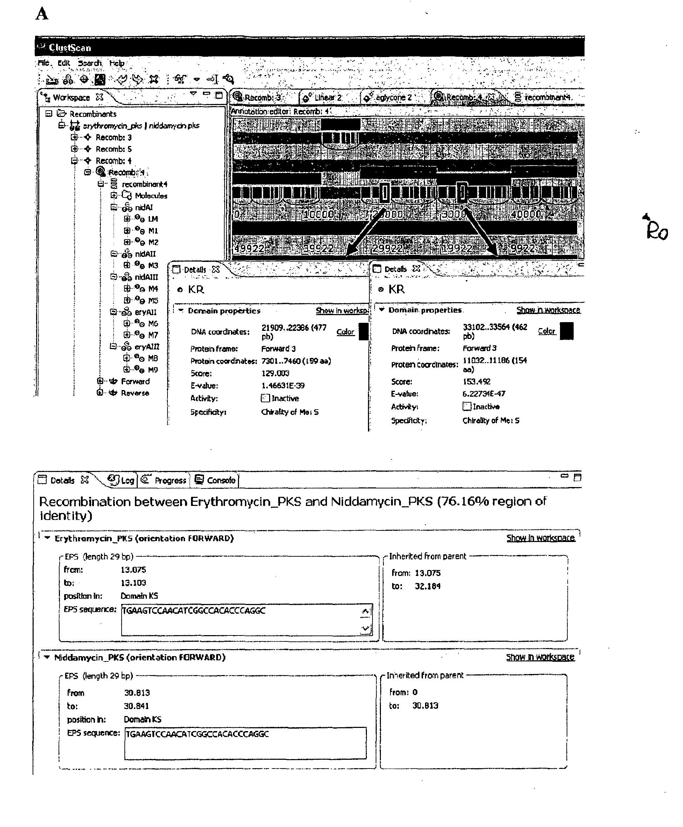

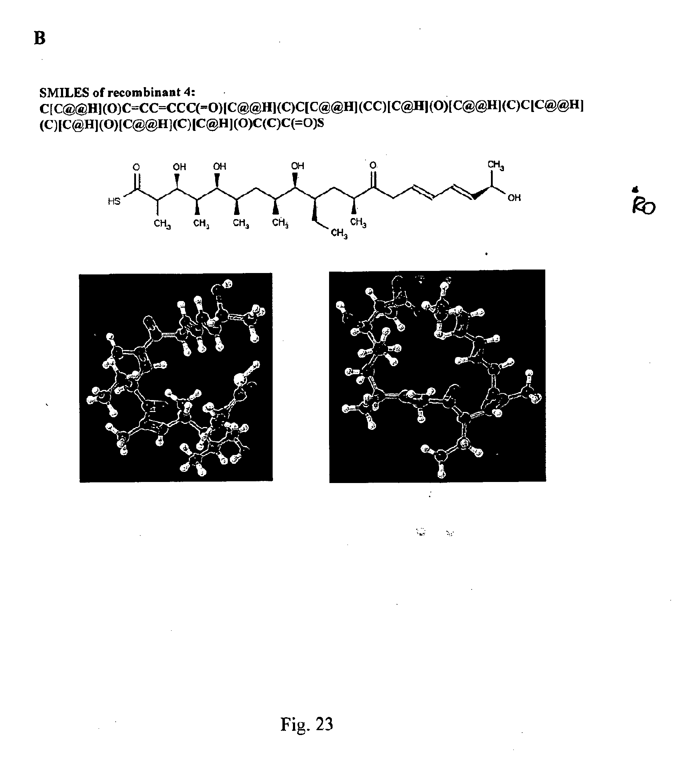

The functional prediction of the DNA sequence of the 4th recombinant between the 14-membered macrolide erythromycin and the 16-membered macrolide niddamycin gene-clusters is shown in Fig. 23A. The method correctly predicts 5 genes (*nid*AI, *nid*AII, *nid*AIII, *ery*AII and *ery*AIII), 5 proteins, 9 modules and 45 catalytically active domains of the fourth recombinant gene-cluster. It also predicts that the loading AT domain loads malonyl-CoA, which was after the initial decarboxylation by the KS^{Q}, was converted to acetyl. The other nine AT domains of the extender modules loads three malonyl-CoA, five methylmalonyl-CoA and one ethylmalonyl-CoA, respectively. The correct predictions of the α-carbon reduction and the α- and β-carbon stereochemistry by the KR's and ER's were also achieved. In particular, the inactive KR in module 4 and the inactive KR in module 6 both responsible for the β-carbon: S stereochemistry were predicted. The linear chain of the 18-membered macrolide and its cyclic aglycon structure were also predicted. The 4th recombinant gene-cluster linear and cyclic products are shown in Fig. 23B.

### Example 8

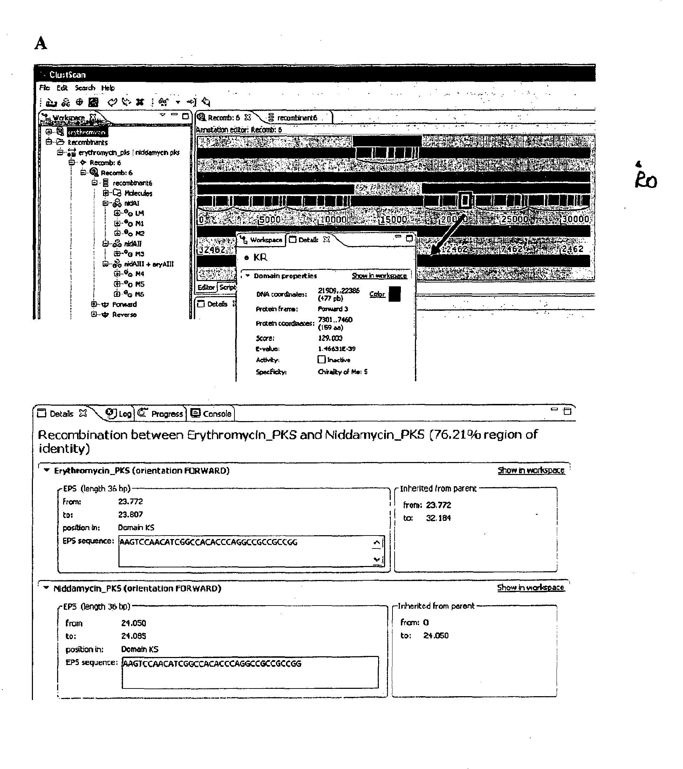

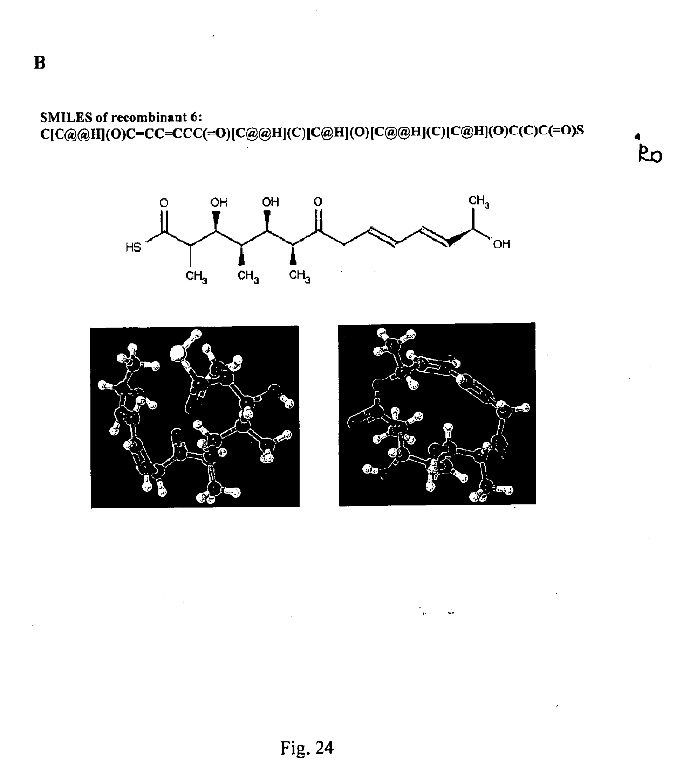

The functional prediction of the DNA sequence of the 6th recombinant between the 14-membered macrolide erythromycin and the 16-membered macrolide niddamycin gene-clusters is shown in Fig. 24A. The method predicts 3 genes (*nid*AI, *nid*AII, *nid*AIII + *ery*AIII), 3 proteins, 6 modules and 30 catalytically active domains of the sixth recombinant gene-cluster. It also predicts that the loading AT domain loads, after the initial decarboxylation by the KS^{Q}, acetyl-CoA while the other six AT domains of the extender modules loads three malonyl-CoA and three methylmalonyl-CoA, respectively. The predictions of the α-carbon reduction and the α- and β-carbons stereochemistry by the KR's and ER's were also achieved. In particular, the inactive KR in module 4 responsible for the β-carbon: S stereochemistry was predicted. The linear chain of the 14-membered macrolide and its cyclic aglycon structure were also predicted. The sixth recombinant gene-cluster linear and cyclic products are shown in Fig. 24B.

## Claims

1. In silico method for the annotation of natural product gene-clusters and for the generation of novel biologically active chemical entities from DNA sequences wherein said method comprising the steps of:
• automatic acquisition, structuring, storage and graphical presentations of data from the publicly available databases for modular biosynthetic pathways for polyketide, non- ribosomal peptide, siderophore, hybrid and other products,
• semi-automatic uploading of the custom database,
• importing of the gene-cluster, genome or metagenome DNA sequence either from the Web, hard disk or the server,
• translation of the DNA sequence into all six open reading frames,
• localisation of genes within the translated protein sequences by GenMark or Glimmer using stringent or relaxed parameters,
• identification of PKS, NRPS, NIS, hybrid and other modular gene-cluster domains within found genes using protein profiles or customised profiles,
• annotation of gene-clusters and biosynthetic pathways by semi-automatic approach,
• exporting final annotation as EMBL, GenBank or XML files for use in other applications and for submission to custom or public databases,
• prediction of putative chemical structures of linear polyketide/peptide/siderophore chains and their upload into the custom database,
where AT domain specificity toward malonyl and methylmalonyl is obtained by comparison with five fingerprints consisting of 13 amino acid residues on position 7, 70, 96, 97, 98, 99, 100, 123, 197, 198, 229, 248, 253, where fingerprints are as follows:
- QQGHS[L,V,I,F,M]GR[F,P]H[A,N,T,G,E,D,S,-][N,H,Q]V for malonyl, and
- QQGHS[Q,M,I]GRSHT[N,S]V for methylmalonyl,
characterise by that the said method further comprises
• generation of recombinant DNA sequences, annotation of gene-clusters of recombinant DNA sequences, prediction of putative linear and cyclic products and their upload into custom database
• the prediction of putative cyclic chemical structures from the linear polyketide/peptide/ siderophore/hybrid and other product chains and their upload into the custom database,
• prediction of biological activities of the *in silico* generated novel chemical entities and their upload into the custom database;
and where seven domains KS, AT, ER, DH, KR, ACP or TE are detected by use of HMMER profiles; where AT domain extender specificity toward three extender substrates - ethylmalonyl, propionyl and methylbutyryl is obtained by comparison with five fingerprints and where if the amino acids did not fit any of the five fingerprints, the AT domain is aligned using a profile derived from the 11 methoxymalonyl-CoA AT domains, wherein fingerprints for three extenders consist of 13 amino acid residues:
- (Q[Q or H]G[H or S]S[Q or L]GR[G or T or A]HTNV) for ethylmalonyl-CoA,
- QQGHS[Q,M,I]GWAH[S,G]SV for propionyl-CoA,
- QQGHS[Q,M,I]GWAH[S,G]NV for methylbutyryl,
and methoxymalonyl incorporating AT domain is defined by existence of at least one of the two insertions having conserved W or P respectively, and where said amino-acid are on the same position as amino acids for determination of specificity At domain toward malonyl and methylmalonyl.

2. Method according to claim 1, wherein prediction is obtained by generating a multiple alignment of newly identified AT with a trusted set of AT's followed by parsing and checking of the said alignment for the key residues in the 2D matrix.

3. Method according to claim 1, wherein activity of the KR domain is predicted by fingerprints comprising a set of 4 critical amino acid residues.

4. Method according to claim 3, wherein the set of 4 critical amino acid residues for the KR activity is (K[S or A or G]YN or E[S or A or G]HH or K[S or A or G]Y[N or G]).

5. Method according to claim 1, wherein a specificity of the KR domain is predicted by fingerprints comprising a set of 8 residues that are mapped and numbered according to the erythromycin KR from the first module of the DEBS1 in context of a multiple alignment of all polyketide KR domains with known activities and specificities.

6. Method according to claim 5, wherein all KR domains having the right combinations of residues when aligned to the trusted set of KR's are grouped as A1 (LDD and W [anything except H]YAN), A2 (LDD and WHYAN), B1 (LDDXXY [anything except P]N), B2 (LDDXXYPN), C1 (XXXXX[anything except Y]XX) or C2 (XXXXXYX[anything except N]).

7. Method according to claim 1, wherein for distinction of active and inactive DH domains is determined by a score created with regard to the profile built using the sequences of 57 active domains derived from actinomycetes where a custom profile was used to screen the active domains used in its construction as well as new 56 active and 46 inactive domains.

8. Method according to claim 7, wherein domains with a score >300 are active, domains with score < 200 are inactive and for domains having scores between 200 and 300, 80% probability of activity is assigned, and the scores of inactive domains were distributed throughout the range.

9. Method according to claim 1, wherein an active ER domain is determined by the existence of active KR and DH domains.

10. Method according to claim 1, wherein for the prediction of putative chemical structures of the linear polyketide/peptide/siderophore chains extender substrates have been organized into the custom database according to their type, reduction degree, stereochemistry and isomerisation.

11. Method according to claim 1, wherein for obtaining cyclic structures, all possible cyclisation points are selected and molecular dynamics simulation is firstly performed for each of these potential structures to get a viable view of active site TE domain-linear chain complex, then potential of the mean force is calculated to assess feasibility of each cyclic structure and finally, structures are sorted depending on how feasible they are.

12. Method according to claim 1, wherein for the purposes of generating recombinant DNA, a Cluster class corresponding closely to the cluster entries in the custom databases is designed, in particular it contains both sequence information and information about module functionality so that the linear synthesis product can be deduced wherein recombination generates the recombinant Cluster objects.

13. Method according to claim 10, wherein the method requires a length of perfect homology, about 27 bp, and similarity of about 75% in the surrounding 200 bp, including identification of chi-sites by screening the genomic sequences of actinomycetes.

14. Method according to claim 1, wherein prediction of biological activity is done by computer aided drug design technology.

15. In silico method for obtaining gene cluster for biosynthesis of chemical compound with given structural formula comprising inversion of steps defined for the method claimed in claim 1-12.

16. A computer program package adapted to carry out the method for the annotation of natural product gene-clusters and for the generation of novel biologically active chemical entities from DNA sequences according to any of claims 1-13 when run on a computer.

17. The computer program package according to the claim 14, capable of browsing or searching the content or using the structure of the custom database of polyketide, non-ribosomal peptide, sideophore, hybrid and other product of modular biosynthetic pathways based on BioSQL v 1.29, wherein the said database contains all the data from the DNA sequence of well described PKS, NRPS, NIS, hybrid and other modular biosynthetic gene cluster, as well as clusters that have been deduced on the basis of DNA sequencing of bacterial genomes and metagenome data sets, and wherein said database contains the description of the biosynthetic process that allows programs to automatically deduce the altered products produced by homologous recombination.

18. A graphical interface for the annotation of natural product gene-clusters and for the generation of novel biologically active chemical entities from DNA sequences according to any of claims 1-13 when run on a computer being designed to enable access to the position of the genes and domains shown on six reading frames, for displacing genes and domains for the purposes of visualisation of overlapping regions, and definition of a cluster covering a region of adjacent genes.

19. The interface according to claim 16, wherein a details window presents evidence for the identification of the domains showing coordinates of the domain in the DNA, protein, scores and E values from the HMMER analysis and alignment ci the protein with the profile, domain functionality and its specificity.

## Patentansprüche

1. In Silico Methode zur Annotation von Genclustern des Naturprodukts und zur Generierung von neuen bioaktiven chemischen Einheiten aus DNA-Sequenzen, wobei die Methode folgende Schritte umfasst:
• automatische Erhebung, Strukturierung, Speicherung und grafische Darstellung der Daten aus öffentlich zugänglichen Datenbanken für modulare, biosynthetische Prozesse für Polyketide, nichtribosomale Peptide, Siderophore, Hybride und andere Produkte,
• halbautomatisches Hochladen der benutzerdefinierten Datenbank,
• Import der Gencluster, DNA-Genom- oder Metagenomsequenz, aus dem Internet, von der Festplatte oder vom Server,
• Translation der DNA-Sequenz in alle sechs offenen Leserahmen,
• Lokalisierung der Gene innerhalb der translatierten Proteinsequenzen mittels GenMark oder Glimmer an Hand stringenter oder relaxierter Parameter,
• Identifikation von PKS, NRPS, NIS, Hybriden und anderen modularen Domänen der Gencluster in den gefundenen Genen an Hand Protein- oder benutzerdefinierter Profile,
• halbautomatische Annotation der Gencluster und biosynthetischen Prozesse,
• Export der finalen Annotation in Form von EMBL, GenBank oder XML Dateien zum Einsatz in anderen Anwendungen sowie zur Übermittlung an benutzerdefinierte oder öffentliche Datenbanken,
• Vorhersage der putativen chemischen Strukturen der linearen Ketten der Polyketide/Peptide/Siderophore und deren Hochladen in eine benutzerdefinierte Datenbank,
wo sich die Spezifität der AT-Domäne zum Malonyl und Methylmalonyl aus Vergleichen mit fünf Fingerabdrücken ergibt, bestehend aus 13 Aminosäureresten an Positionen 7, 70, 96, 97, 98, 99, 100, 123, 197, 198, 229, 248, 253, wo die Fingerabdrücke wie folgt sind:
- QQGHS[L,V,I,F,M]GR[F,P]H[A,N,T,G,E,D,S,-][N,H,Q]V für Malonyl, und
- QQGHS[Q,M,I]GRSHT[N,S] für Methylmalonyl,
**gekennzeichnet dadurch, dass** die genannte Methode des weiteren Folgendes umfasst:
• Generierung der rekombinanten DNA-Sequenzen, Annotation der Gencluster der rekombinanten DNA-Sequenzen, Vorhersage der putativen linearen und zyklischen Produkte und deren Hochladen in eine benutzerdefinierte Datenbank,
• Vorhersage der putativen zyklischen chemischen Strukturen aus linearen Ketten der Polyketide/Peptide/Siderophore/Hybride und Ketten anderer Produkte sowie deren Hochladen in eine benutzerdefinierte Datenbank,
• Vorhersage der Bioaktivitäten der In Silico generierten neuen chemischen Einheiten und deren Hochladen in eine benutzerdefinierte Datenbank;
und wo die sieben Domänen (KS-, AT-, ER-, DH-, KR-, ACP- oder TE-Domäne) mittels HMMER Profilen detektiert werden; wo sich der Extender der Spezifität der AT-Domäne zu den drei Extendern der Substrate - Ethylmalonyl, Propionyl und Methylbutyryl aus Vergleichen mit fünf Fingerabdrücken ergibt, wobei, wenn die Aminosäuren keinem der fünf Fingerabdrücke entsprechen, die AT-Domäne mittels Profil aligniert wird, das aus 11 Methoximalonyl-CoA Domänen abgeleitet wird, wobei die Fingerabdrücke für drei Extender aus 13 Aminosäureresten bestehen:
- (Q[Q oder H]G[H oder S]S[Q oder L]GR[G oder T oder A]HTNV) für Ethylmalonyl-CoA,
- QQGHS[Q, M, I]GWAH[S, G] für Propionyl-CoA,
- QQGHS[Q, M, I]GWAH[S, G]NV für Methylbutyryl,
und das die AT-Domäne umfassende Methoximalonyl definiert sich mit Vorhandensein von mindestens einer von zwei Insertionen, die jeweils W oder P erhalten haben, und wo sich die genannten Aminosäuren an derselben Position zur Bestimmung der Spezifität der AT-Domäne zu Malonyl oder Methylmalonyl befinden.

2. Methode gemäß Anspruch 1, wobei die Vorhersage mittels Generierung eines multiplen Alignments der neu identifizierten AT mit einem zuverlässigen AT-Satz erfolgt, wonach Parsing und Nachprüfung des genannten Alignments für die wichtigsten Reste in der 2D-Matrix stattfindet.

3. Methode gemäß Anspruch 1, wobei die Aktivität der KR-Domäne an Hand Fingerabdrücke vorhergesagt wird, die einen Satz aus vier entscheidenden Aminosäureresten umfassen.

4. Methode gemäß Anspruch 3, wobei der Satz aus vier entscheidenden Aminosäureresten für die KR-Aktivität (K[S oder A oder G]YN oder E[S oder A oder G]HH oder K[S oder A oder G]Y[N oder G] ist.

5. Methode gemäß Anspruch 1, wobei eine Spezifität der KR-Domäne an Hand Fingerabdrücke vorhergesagt wird, die einen Satz aus acht Resten umfassen; diese sind entsprechend KR-Erythromyzin aus dem ersten DEBSI-Modul im Kontext eines multiplen Alignments aller KR-Domänen der Polyketide mit bekannten Aktivitäten und Speziftäten gemappt und numeriert.

6. Methode gemäß Anspruch 5, wobei alle KR-Domänen mit richtigen Kombinationen der Reste, wenn sie zu einem zuverlässigen KR-Satz aligniert sind, als A1 (LDD i W [alles außer H]YAN), A2 (LDD und WHYAN), B1 (LDDXXY) [alles außer P]N), B2 (LDDXXYPN), C1 (XXXXX [alles außer Y]XX) oder C2 (XXXXXYX [alles außer N]) gruppiert sind.

7. Methode gemäß Anspruch 1, wobei der Unterschied zwischen aktiven und inaktiven DH-Domänen durch den Score bestimmt ist, der in Bezug auf das Profil kreiert wird, das aus Sequenzen von 57 aktiven, aus Aktinomyzeten abgeleiteten Domänen besteht, wobei ein angepasstes Profil zum Screen der bei seinem Bau eingesetzten aktiven Domänen sowie neue 56 aktive und 46 inaktive Domänen verwendet werden.

8. Methode gemäß Anspruch 7, wobei die Domänen mit dem Score >300 aktiv, und Domänen mit dem Score <200 inaktiv sind; die Aktivität der Domänen mit dem zwischen 200 und 300 liegenden Score wird mit 80%-iger Wahrscheinlichkeit angesetzt, und die Scores der inaktiven Domänen sind über den gesamten Bereich distribuiert.

9. Methode gemäß Anspruch 1, wobei die aktive ER-Domäne durch Vorhandensein der aktiven KR- und DH-Domänen bestimmt ist.

10. Methode gemäß Anspruch 1, wobei zur Vorhersage der putativen chemischen Strukturen der linearen Ketten der Polyketide/Peptide/Siderophore die Extendersubstrate in einer benutzerdefinierten Datenbank nach Typ, Reduktionsgrad, Stereochemie und Isomerisation organisiert sind.

11. Methode gemäß Anspruch 1, wobei zur Gewinnung der zyklischen Strukturen alle möglichen Zyklisierungspunkte genommen werden, und für jede dieser möglichen Strukturen zunächst eine molekulare Dynamik simuliert wird, und zwar zur Gewinnung einer brauchbaren Sicht des Komplexes TE-Domäne-lineare Kette an aktiver Position; anschließend wird das Potenzial der mittleren Kraft zur Einschätzung der Feasibility jeder der zyklischen Strukturen ermittelt, und schließlich werden die Strukturen abhängig von ihrer Feasibility sortiert.

12. Methode gemäß Anspruch 1, wobei zur Generierung der rekombinanten DNA eine Clusterkategorie kreiert wird, die mit den Clustereintragungen in die benutzerdefinierten Datenbanken in enger Übereinstimmung steht, und insbesondere sowohl Sequenzinformation als auch Information über die Funktionalität des Moduls enthält, so dass das Produkt der linearen Synthese abgeleitet werden kann, wobei die Rekombination rekombinante Clusterobjekte generiert.

13. Methode gemäß Anspruch 10, wobei die Methode Länge einer perfekten Homologie erfordert, rd. 27 bp, und die Ähnlichkeit von rd. 75% in den umliegenden 200 bp, einschließlich Identifikation der Chi-Sites mittels Screening der Genomsequenzen der Aktinomyzeten.

14. Methode gemäß Anspruch 1, wobei die Vorhersage der Bioaktivitäten mittels computerunterstützter Arzneimittelentwicklungstechnologie erfolgt.

15. In Silico Methode zur Gewinnung von Genclustern zur Biosynthese der chemischen Verbindung mit gegebener Strukturformel, die die Inversion der für die Methode gemäß Anspruch 1-12 definierten Schritte umfasst.

16. Software-Paket zur Anwendung der Methode zur Annotation der Gencluster des Naturprodukts und zur Generierung von neuen bioaktiven chemischen Einheiten aus DNA-Sequenzen gemäß einem der Ansprüche 1-13, wenn die Aktivität am Computer stattfindet.

17. Software-Paket gemäß Anspruch 14 zum Durchsuchen der Inhalte oder zur Verwendung der Struktur der benutzerdefinierten Datenbank der Polyketide, nichtribosomale Peptide, Siderophore, Hybride und anderen Produkte modularer, biosynthetischer, auf BioSQL v 1.29 basierender Prozesse, wobei die genannte Datenbank alle Daten der DNA-Sequenz der gut beschriebenen PKS, NRPS, NIS, Hybride und anderen biosynthetischen Gencluster sowie Cluster enthält, die an Hand der DNA-Sequenzierung der Bakteriengenome und Metagenomdatensätze abgeleitet sind, und wobei die genannte Datenbank eine Beschreibung des Biosyntheseverfahrens enthält, die es dem Programm möglich macht, die durch homologe Rekombination hergestellten Produkte automatisch abzuleiten.

18. Grafikschnittstelle zur Annotation von Genclustern des Naturprodukts und zur Generierung von neuen bioaktiven chemischen Einheiten aus DNA-Sequenzen gemäß einem der Ansprüche 1-13, wenn sie am Computer stattfinden, ausgestaltet zur Gewährung des Zugangs zur Position der Gene und Domänen, die in sechs Leserahmen dargestellt sind, zur Verschiebung von Genen und Domänen zu Zwecken der Visualisierung der Überlappungen und zur Definition des Clusters, das den Bereich der benachbarten Gene umfasst.

19. Schnittstelle gemäß Anspruch 16, wobei das Detailfenster ein Nachweis zur Identifikation der Domänen darstellt und Koordinaten der Domänen in der DNA, Protein, Scores und E-Werte aus der HMMER-Analyse sowie Alignment des Ci Proteins mit dem Profil, der Funktionalität der Domäne und deren Spezifität zeigt.

## Revendications

1. Un procédé *in silico* pour l'annotation de groupes de gènes de produit naturel et pour la génération de nouvelles entités chimiques biologiquement actives à partir de séquences d'ADN, ledit procédé comprenant les étapes suivantes :
• l'acquisition, structuration, stockage et représentation graphique automatiques des données depuis des bases de données publiques des voies biosynthétiques modulaires pour polycétides, peptides non ribosomiques, sidérophores, hybrides modulaires et autres produits,
• chargement semi-automatique d'une base de données spécifique sur mesure,
• importation du groupe de gènes et de la séquence d'ADN génomique ou métagénomique à partir d'un site Web, d'un disque dur ou d'un serveur,
• traduction du séquençage de l'ADN dans les six cadres de lecture ouverts,
• localisation des gènes au sein des séquences protéiques traduites (programmes de détection GenMark ou Glimmer) à l'aide de paramètres stricts ou moins stricts,
• identification des domaines modulaires des groupes de gènes PKS, NRPS, NIS, hybrides et autres au sein des gènes trouvés à l'aide de profils protéiques ou profils spécifiquement adaptés,
• annotation par approche semi-automatique des groupes de gènes et des voies de biosynthèse,
• exportation des annotations finales sous forme de fichiers de type EMBL, GenBank ou XML pouvant être utilisés par d'autres applications et pouvant être translocalisés dans des bases de données publiques ou sur mesure,
• prédiction des structures chimiques putatives des chaînes linéaires polycétides/peptides/sidérophores et leur chargement dans la base de données sur mesure,
où la spécificité du domaine AT envers malonyle et méthylmalonyl est obtenue en comparant cinq empreintes digitales composées de 13 résidus d'acides aminés aux emplacements numérotés 7, 70, 96, 97, 98, 99, 100, 123, 197, 198, 229, 248, 253 et où les empreintes digitales se présentent comme suit :
- QQGHS [L, V, I, F, M] GR [F, P] H [A, N, T, G, E, D, S, -] [N, H, Q] V pour le groupe malonyle et
- QQGHS [Q, M, I] GRSHT [N, S] pour le groupe méthylmalonyl,
**caractérisé en ce que** le procédé ci-dessus indiqué inclut également :
• la génération de séquences d'ADN recombinant, l'annotation des groupes de gènes des séquences d'ADN recombinant, la prédiction de produits linéaires et cycliques putatifs et leur chargement dans la base de données sur mesure,
• la prédiction de structures chimiques cycliques putatives issues des chaînes linéaires polycétides/peptides/sidérophores/hybrides et autres chaînes de produits ainsi que leur chargement dans la base de données sur mesure,
• la prédiction d'activités biologiques *in silico* des nouvelles entités chimiques générées et leur chargement dans la base de données sur mesure ;
où les sept domaines KS, AT, ER, DH, KR, ACP ou TE sont détectés à l'aide de profils HMMER ; où la spécificité de l'extendeur du domaine AT envers trois substrats de l'extendeur (éthylmalonyl, propionyl et méthylbutyryl) est obtenue par comparaison avec cinq empreintes digitales et où, au cas où les acides aminés ne correspondraient à aucune des cinq empreintes digitales, le domaine AT est aligné au moyen d'un profil issu des 11 domaines AT méthoxymalonyl-CoA et où les empreintes digitales pour trois extendeurs comprennent 13 résidus d'acides aminés :
- (Q[Q ou H]G[H ou S]S[Q ou L]GR[G ou T ou A]HTNV) pour éthylmalonyl-CoA,
- QQGHS[Q, M, I]GWAH[S, G] pour propionyl-CoA,
- QQGHS[Q, M, I]GWAH[S, G]NV pour méthylbutyryl,
et où le méthoxymalonyl comprenant le domaine AT est défini par l'existence d'au moins une des deux insertions ayant conservé soit W soit P et où lesdits acides aminés se trouvent au même endroit que les acides aminés servant à déterminer la spécificité du domaine AT envers malonyle et méthylmalonyl.

2. Un procédé selon la demande 1 où la prédiction est obtenue par la génération d'un alignement multiple de l'AT nouvellement identifié avec une série fiable d'AT, suivi de l'analyse et de la vérification dudit alignement en ce qui concerne les principaux résidus dans la matrice 2D.

3. Un procédé selon la demande 1 où l'activité du domaine KR se prévoit à l'aide d'empreintes digitales comprenant un groupe de 4 résidus clés d'acides aminés.

4. Un procédé selon la demande 3 où le groupe de 4 résidus clés d'acides aminés pour l'activité du domaine KR est (K[S ou A ou G]YN ou E[S ou A ou G]HH ou K[S ou A ou G]Y[N ou G].

5. Un procédé selon la demande 1 où la spécificité du domaine KR se prévoit à l'aide d'empreintes digitales comprenant un groupe de 8 résidus cartographiés et numérotés en fonction de l'érythromycine KR du premier module DEBSI dans un contexte d'alignement multiple de tous les domaines KR de polycétides aux activités et spécificités connues.

6. Un procédé selon la demande 5 où tous les domaines KR présentant la bonne combinaison de résidus lorsqu'ils sont alignés avec un groupe de KR fiables sont regroupés en tant que A1 (LDD et W [tout sauf H]YAN), A2 (LDD et WHYAN), B1 (LDDXXY) [tout sauf P]N), B2 (LDDXXYPN), C1 (XXXXX [tout sauf Y]XX) ou C2 (XXXXXYX [tout saufN]).

7. Un procédé selon la demande 1 où la différence entre domaines DH actifs et non actifs est déterminée par le résultat créé en fonction d'un profil construit avec les séquences de 57 domaines actifs dérivés d'actinomycètes et où l'on s'est servi d'un profil adapté permettant le criblage des domaines actifs ayant été utilisés pour sa construction ainsi que celui de 56 nouveaux domaines actifs et 46 nouveaux domaines non actifs.

8. Un procédé selon la demande 7 où les domaines dont le résultat est >300 sont actifs, ceux avec un résultat <200 sont non actifs et où une probabilité de 80 % d'activité est attribuée aux domaines ayant un résultat compris entre 200 et 300 et où les résultats des domaines non actifs sont répartis à travers toute la gamme.

9. Un procédé selon la demande 1 où le domaine actif ER est déterminé par l'existence de domaines actifs KR et DH.

10. Un procédé selon la demande 1 où, pour la prédiction de structures chimiques putatives de chaînes linéaires polycétides/peptides/sidérophores, les substrats d'extendeur ont été organisés dans la base de données sur mesure en fonction de leur type, de leur potentiel de réduction, de leur stéréochimie et de leur isomérisation.

11. Un procédé selon la demande 1 où, pour l'obtention de structures cycliques, tous les points possibles de cyclisation sont sélectionnés et où, pour chacune de ces structures potentielles, une simulation par dynamique moléculaire est réalisée au préalable afin d'obtenir un aperçu viable du complexe de la chaîne domaine-linéaire TE en région active, puis le potentiel de force moyenne est calculé en vue d'évaluer l'éventualité de chacune de ces structures cycliques puis ces structures sont classées en fonction de leur éventualité.

12. Un procédé selon la demande 1 où, en vue de générer un ADN recombinant, le groupe de gènes est placé dans une catégorie étroitement liée aux groupes de gènes saisis dans la base de données sur mesure et contenant en particulier les informations sur la séquence et celles relatives à la fonctionnalité du module afin de déterminer le produit de la synthèse linéaire, la recombinaison générant des objets recombinants de groupes de gènes.

13. Un procédé selon la demande 10 exigeant une longueur d'homologie parfaite, d'environ 27 pb, et une similarité d'environ 75 % des 200 pb environnantes, et comprenant l'identification de séquences de Chi par criblage des séquences génomiques d'actinomycètes.

14. Un procédé selon la demande 1 où la prédiction d'activité biologique est obtenue grâce à la technologie de conception de médicaments assistée par ordinateur.

15. Un procédé *in silico* pour l'obtention de groupes de gènes pour la biosynthèse d'un composé chimique de formule structurale donnée incluant l'inversion des étapes définies pour le procédé selon les demandes de 1 à 12.

16. Un logiciel informatique permettant la réalisation du procédé d'annotation de groupes de gènes de produit naturel ainsi que la génération de nouvelles entités chimiques issues d'un séquençage d'ADN selon l'une ou l'autre des demandes de 1 à 13, si ledit procédé est conduit sur ordinateur.

17. Un logiciel informatique selon la demande 14 pouvant naviguer ou rechercher un contenu ou utiliser une structure adaptée à la base de données sur mesure des processus biosynthétiques modulaires de polycétides, peptides non ribosomiques, sidérophores, hybrides modulaires et autres produits se basant sur BioSQL v 1.29, où ladite base de données contient toutes les données de séquençage d'ADN de PKS, NRPS, NIS, hybrides et autres groupes de gènes modulaires biosynthétiques déjà bien décrits ainsi que de groupes de gènes déduits sur la base d'un séquençage de l'ADN de génomes bactériens et de séries de données métagénomiques et où ladite base de données contient une description du processus biosynthétique permettant au programme de tirer des conclusions automatiques relatives aux produits modifiés obtenus par recombinaison homologue.

18. Une interface graphique pour l'annotation des groupes de gènes de produit naturel et pour la génération de nouvelles entités chimiques biologiquement actives issues d'un séquençage d'ADN conformément à certaines des demandes de 1 à 13 lorsque réalisées sur un ordinateur et permettant l'accès à la position des gènes et des domaines indiqués dans les six cadres de lecture ainsi que le déplacement des gènes et des domaines afin de visualiser les zones de chevauchement et de définir un groupe de gènes recouvrant une zone de gènes adjacents.

19. Une interface selon la demande 16 où un affichage détaillé met en évidence l'identification des domaines montrant les coordonnées du domaine dans l'ADN, la protéine, les résultats et les valeurs E résultant de l'analyse HMMER ainsi que l'alignement de la protéine Ci avec profil et fonctionnalité du domaine ainsi que sa spécificité.
